# EUROPEAN PATENT SPECIFICATION

(11) **EP 2 667 863 B1**
(45) Date of publication and mention of the grant of the patent: **30.11.2016**
(21) Application number: 12702865.2
(22) Date of filing: 26.01.2012
(51) Int. Cl.: A61K 31/121, A61P 23/02

(54) **LOCAL ANAESTHETIC**
LOKALANÄSTHETIKUM
ANESTHÉSIQUE LOCAL

(30) Priority: 27.01.2011 GR 20110100037; 11.02.2011 GB 201102411; 11.02.2011 US 201161441940 P
(43) Date of publication of application: 04.12.2013
(73) Proprietor: Vita (Europe) Limited, Basingstoke Hampshire RG21 7PG (GB)
(72) Inventor: WATKINS, Max, Basingstoke Hampshire RG21 7PG (GB); THEOPHILIDIS, George, GR-54 124 Thessaloniki (GR); PAPACHRISTOFOROU, Alexandros, GR-54 124 Thessaloniki (GR)
(74) Representative: D Young & Co LLP
(86) International application number: PCT/GB2012/050157
(87) International publication number: WO 2012/101440

(56) References cited:
- WON, A. ET AL.: "The minimum alveolar anesthetic concentration of 2-, 3-, and 4-alcohols and ketones in rats: relevance to anesthetic mechanism", ANESTH ANALG, vol. 102, 2006, pages 1419-1426, XP002672673,

## Description

### FIELD

The present invention relates to the field of anaesthesia, and in particular to compounds and compositions having a local anaesthetic action, especially in mammals.

### BACKGROUND

Local anaesthesia refers to a state in which a region of the body is insensitive to pain, typically whilst the subject remains conscious. It is often used clinically during surgical and dental procedures, in order to reduce or eliminate the pain experienced by the subject. Thus the anaesthetized region may be, for example, an area of skin or a tooth. It is typically preferable to use local anaesthesia rather than general anaesthesia where possible, in order to avoid the increased monitoring required and risk to the subject associated with general anaesthesia.

Local anaesthetics typically act by inhibiting sodium influx through the neuronal cell membrane. In particular, most known local anaesthetics block voltage-gated sodium channels, which are involved in conducting action potentials along nerve fibres. Local anaesthetics drugs usually bind preferentially with activated sodium channels, resulting in increased selectivity for rapidly firing neurons.

One of the most commonly used local anaesthetics is lidocaine (lignocaine, 2-(diethylamino)-N-(2,6-dimethylphenyl)-acetamide). Lidocaine is a weak base, and is frequently employed as its hydrochloride salt to improve its water solubility. Local anaesthetics such as lidocaine are usually administered by injection into the area of the nerve fibres to be blocked. Alternatively, some local anaesthetics may be applied topically, in which case the drug must diffuse through an intact skin barrier to exert its anaesthetic effect. Lidocaine, in addition to its widespread use as a local anaesthetic, may also be used in the treatment of ventricular tachycardia as an intravenous injection solution, and in the treatment of various types of nerve pain (neuralgia). However, one of the limitations of many local anaesthetics such as lidocaine is their reported neurotoxicity (see e.g. Perez-Castro et al., Anesthesia& Analgesia (2009), 108(3):997-1007).

Whilst various local anaesthetic compounds are known, there is still a need for further local anaesthetics with improved properties. In particular, there is a need for a simple, safe and effective anaesthetic that is long lasting, non-toxic and can easily be administered and produced.

Won et al, Anesth Analg. 2006 May;102(5):1419-26 describes the minimum alveolar anesthetic concentration of 2-, 3-, and 4-alcohols and ketones in rats and the relevance to anesthetic mechanisms.

### SUMMARY

Accordingly, in one aspect the present invention provides heptanone (e.g. 2-, 3- or 4-heptanone) or a salt thereof for use as a local anaesthetic.

In a further aspect, the invention provides a pharmaceutical composition comprising heptanone (e.g. 2-, 3- or 4-heptanone) or a salt thereof and one or more pharmaceutically acceptable carriers, excipients or diluents for use as a local anaesthetic. The composition may be for administration by various routes, such as by injection (e.g. intramuscular or subcutaneous) or for topical application.

In one embodiment, the composition is in the form of a liquid, preferably an aqueous solution. For instance the composition may be formulated as a saline solution. Preferably the composition further comprises a buffer, and is typically sterile.

In one embodiment, the concentration of heptanone (e.g. 2-, 3- or 4-heptanone, for instance in a liquid pharmaceutical formulation) is from 10 mM to 30 mM. For instance, for 2-heptanone the concentration may be about 10 mM (1.13 mg/mL), about 12.5 mM (1.43mg/mL), about 15.0 mM (1.7mg/mL), about 20.0 mM (2.26 mg/mL) or about 30.0 mM (3.4 mg/mL). 3.4 mg/mL is below the solubility limit of 2-heptanone in water which is 4.3 mg/mL. For 3- and 4-heptanone, the concentration may be, for example about 3.4 mg/mL.

In a further aspect, the present invention provides use of heptanone (e.g. 2-, 3- or 4-heptanone) or a salt thereof in the preparation of a medicament for use as a local anaesthetic.

The present inventors have compared the action of heptanone and lidocaine, the standard local anaesthetic, using *ex vivo* methodology (the isolated sciatic nerve of the rat). It has been demonstrated that at 3.4 mg/mL of either compound has the same mode of action, i.e. full inhibition and recovery of nerve activity. There is a delay of only 70-75 seconds in the inhibitory effect of 2-heptanone on the nerve CAP compared to lidocaine; for 3.4mg/mL 2-heptanone the IT₀ is 221.88 seconds and for lidocaine the IT₀ is 148.1 seconds. Accordingly, heptanone has been shown to have a local anaesthetic action similar to that of lidocaine.

### BRIEF DESCRIPTION OF THE DRAWINGS

Figure 1 shows a three-chamber recording bath as used in the Examples. The three chambers (the stimulating chamber, the recording chamber and the perfusion chamber), the recording and the stimulating electrodes, the nerve and the cover and the thin layer of dental paste are shown. 2-heptanone was diluted in the perfusion chamber.
Figure 2 shows the compound action potential (CAP) as a result of the nerve fibre activation or inactivation. The CAP amplitude depends on the number of the active (i.e. producing an action potential) nerve fibres in the sciatic nerve (see cross section). The decrease of the amplitude is an indication of the inactivation of a population of nerve fibres in the presence of 2-heptanone or lidocaine.
Figure 3 shows compound action potential amplitude in rat sciatic nerves after incubation within 30 mM (3.4 mg/mL) 2-heptanone.
Figure 4 shows recovery of the compound action potential in rat sciatic nerves after inhibition by 30 mM 2-heptanone was removed by washing.
Figure 5 shows the reduction in compound action potential in rat sciatic nerves in the presence of 15 mM (1.7 mg/mL) 2-heptanone. Stagnant saline (not stirred).
Figure 6 shows the elimination of the compound action potential in rat sciatic nerves in the presence of 15 mM (1.7 mg/mL) 2-heptanone, wherein the saline solution was stirred continuously.
Figure 7 shows recovery of the CAP of rat sciatic nerve after the washout of 15.0 mM 2-heptanone.
Figure 8 shows the recovery of the CAP of rat sciatic nerves after exposure to 15.0 mM 2-heptanone, wherein the saline solution was stirred continuously.
Figure 9 shows the decrease in the amplitude of the evoked CAP of the rat sciatic nerve of the rat after the incubation of the nerve in 10.0 mM 2-heptanone (stagnant saline).
Figure 10 shows the decrease in the amplitude of the evoked CAP of the rat sciatic nerve of the rat after the incubation of the nerve in 10.0 mM 2-heptanone, wherein the saline solution was stirred continuously.
Figure 11 shows recovery of the CAP of rat sciatic nerve after the washout of 10.0 mM (1.13 mg/mL) 2-heptanone.
Figure 12 shows the recovery of the CAP of rat sciatic nerves after exposure to 10 mM 2-heptanone, wherein the saline solution was stirred continuously.
Figure 13 shows the decrease in the amplitude of the evoked CAP of the rat sciatic nerve after the incubation of the nerve in 10.0 mM (2.35mg/mL) lidocaine.
Figure 14 shows the recovery of the CAP of the rat sciatic nerve after the removal of 10.0 mM (2.35mg/mL) lidocaine.

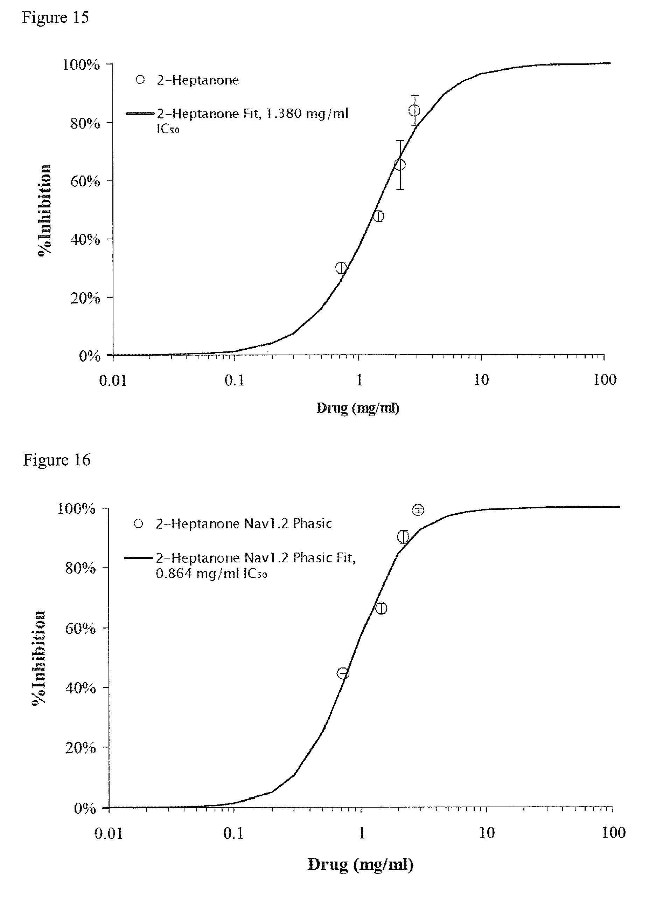

Figure 15 shows a concentration-response curve of the tonic inhibition of Nav1.2 sodium channels expressed in CHO cells by 2-heptanone (n=2).
Figure 16 shows a concentration-response curve of the phasic inhibition of Nav1.2 sodium channels expressed in CHO cells by 2-heptanone (n=2).

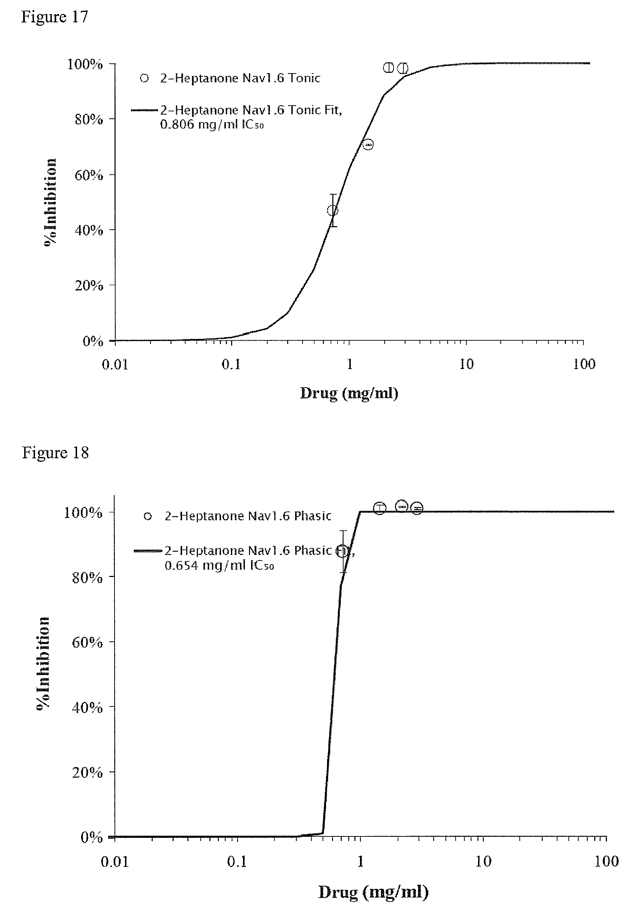

Figure 17 shows a concentration-response curve of the tonic inhibition of Nav1.6 sodium channels expressed in CHO cells by 2-heptanone (n=2).
Figure 18 shows a concentration-response curve of the phasic inhibition of Nav1.6 sodium channels expressed in CHO cells by 2-heptanone (n=2).

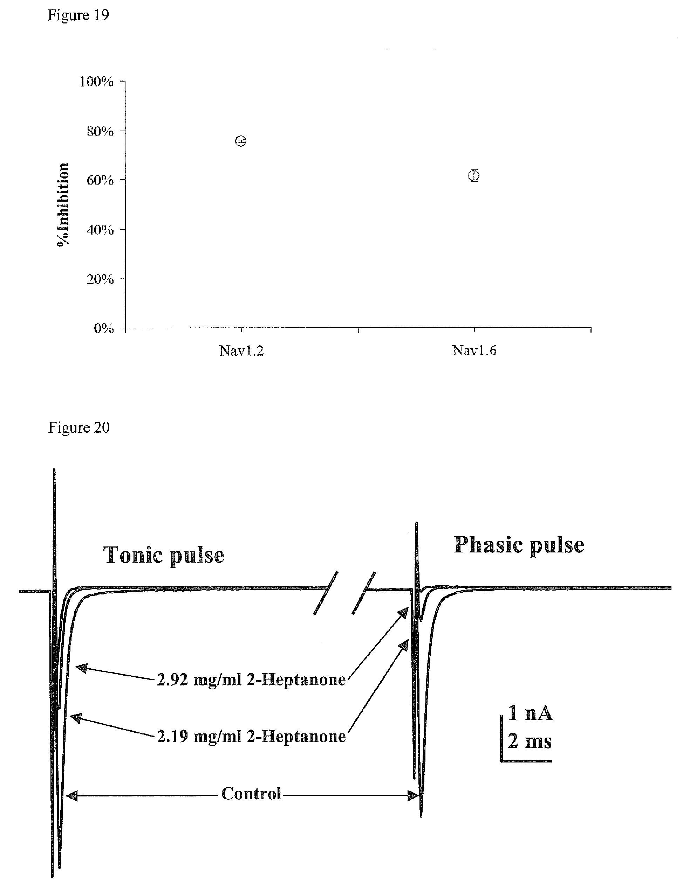

Figure 19 shows the percentage inhibition of Nav1.2 and Nav1.6 sodium channels expressed in CHO cells by 2000 µM (2 mM) lidocaine (n=2).
Figure 20 shows sample patch clamp traces (tonic and phasic pulses) showing the inhibition of individual sodium currents in Nav1.2 cells in the presence of 2.19 or 2.92 mg/ml 2-heptanone.
Figure 21 shows a photograph of the rat preparation for *in vivo* studies used to assess and compare the local anesthetic action of 2-heptanone and lidocane. The stimulating electrode was placed on the sciatic nerve (see arrow Stimulating), while the recording electrodes were injected in the gastrocnemius or biceps muscle to record electromyograms (EMG) (see second arrow, Recording). 2-heptanone and lidocaine were applied with intramuscular injection.
Figure 22 shows the decrease in the amplitude of the nerve compound action potential (CAP) in rat sciatic nerves during incubation in 30.0 mM (3.4 mg/mL) 2-heptanone. The compound was diluted in the saline (solubility of 2-heptanone in water is 4.3 mg/mL) (n=12).
Figure 23 shows the recovery of the evoked CAP of the rat sciatic nerves after washout of the nerve preparation. 30.0 mM 2-heptanone was removed by washing the nerve using normal saline in the perfusion chamber. After washout the recovery of the nerve CAP in normal saline was monitored for 24 h (n=12).
Figure 24 shows the decrease of the nerve CAP during incubation of the rat sciatic nerves in 20.0 mM 2-heptanone (2.28 mg/mL). The compound was diluted in the saline of the perfusion chamber (solubility of 2-heptanone in water is 4.3 mg/mL) (n=4).
Figure 25 shows the recovery of the evoked nerve CAP of the sciatic nerves after the washout of 20.0 mM (2.28 mg/mL) 2-heptanone. After washout, the recovery of the nerve CAP in normal saline was monitored for 24 h (n=4).
Figure 26 shows the elimination of the nerve CAP of the sciatic nerves during incubation in 12.5 mM (1.43 mg/mL) 2-heptanone (n=4).
Figure 27 shows the recovery of the evoked CAP of rat sciatic nerve after the washout of 12.5 mM 2-heptanone. The recovery of the nerve CAP in normal saline was monitored for 24 h after washout (n=4).
Figure 28 shows the decrease in the amplitude of the evoked CAP of the sciatic nerve during incubation of the nerve in 3.4 mg/mL (14.5 mM) (curve a, n=12) and 1.46 mg/mL (6.0 mM) (curve b, n=6) lidocaine.
Figure 29 shows the recovery of the nerve CAP of the sciatic nerve after the washout of 3.4 mg/mL and 1.46 mg/mL lidocaine (curves a,b). The nerve was washed and bathed in normal saline. The nerve CAP was monitored for 24 h (n=12 and 6).
Figure 30 shows: **(A)** Comparing the time-response curves (vitality curves) of the sciatic nerve treated with 3.4 mg/mL 2-heptanone (curve a) and lidocaine (curve b) using the nerve CAP (n=12). **(B)** Comparing the decrease in the amplitude of the evoked CAP of the rat sciatic nerve during the incubation in 10 mg/mL of four known local anaesthetics. Curves (a, b, c, d): lidocaine, mevipacaine, aticaine, prilocaine; curve (e): 2-heptanone (n=7).
Figure 31 shows a comparison of the recovery of the CAP of the rat sciatic nerve after the washout of 3.4 mg/mL 2-heptanone and lidocaine. After the washout the nerve was bathed in normal saline and the amplitude of the nerve CAP was monitored for 24 h (n=12).

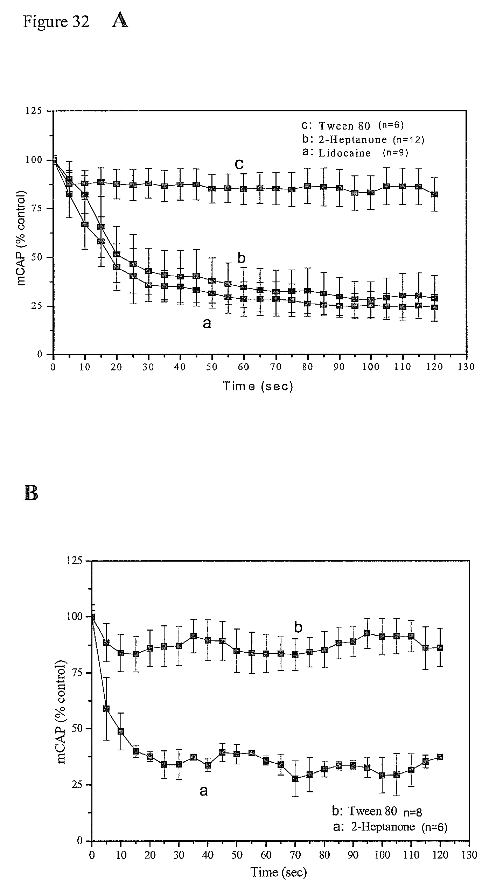

Figure 32 shows **A.** the decrease in the amplitude (integral) of the electromyograms recorded *in-vivo* from the gastrocnemious muscle after intramuscular injection of 10.8 mg/kg lidocaine (curve a, n=9) and 7.2 mg/kg 2-heptanone (curve b, n=12). 2-heptanone was diluted in 139 microL of Tween 80. This amount of Tween 80 was injected as control (curve c, n=6). **B.** the decrease in the amplitude (integral) of the electromyograms recorded *in vivo* from the biceps muscle after intramuscular injection of 7.2 mg/kg 2-heptanone diluted in 139 microL of Tween 80. This amount of Tween 80 was injected as control (curve b).
Figure 33 shows the decrease in the amplitude of the evoked CAP of the rat sciatic nerve after the incubation of the nerve in 3.4 mg/mL of 4-heptanone (n=8).
Figure 34 shows the recovery of the nerve CAP after the removal of 3.4 mg/mL 4-heptanone (n=8). The nerve was washed in normal saline and the CAP was monitored in normal saline for 24 h.
Figure 35 shows the decrease in the amplitude of the evoked nerve CAP of the rat sciatic nerve after the incubation of the nerve in 3.4 mg/mL of 2-hexanone (n=7).
Figure 36 shows the recovery of the CAP in the rat sciatic nerve after the removal of 3.4 mg/mL 2-hexanone. The nerve was washed in normal saline and the CAP was monitored in normal saline for 24 h (n=7).
Figure 37 shows the decrease in the amplitude of the evoked CAP of the rat sciatic nerve after the incubation of the nerve in 3.4 mg/mL of 2-octanone. The 2-octanone was diluted in DMSO, which when was tested alone had no effect on the nerve CAP for over 2h of incubation (n=4).
Figure 38 shows the recovery of the CAP of the rat sciatic nerve after the removal of 3.4 mg/mL 2-octanone. The nerve was washed in normal saline and the nerve CAP was monitored for 24 h (n=4).
Figure 39 (A) shows the decrease in the amplitude of the evoked CAP of the rat sciatic nerve during incubation in 3.4 mg/mL of 3-heptanone (n=6). (B) The recovery after washout of the CAP of nerves exposed to 3-heptanone. The nerve was washed in normal saline and the CAP was monitored in normal saline for 24 h (n=6).
Figure 40 (A) shows the decrease in the amplitude of the evoked CAP of the rat sciatic nerve during incubation 3.4 mg/mL of 2-pentanone (n=6). (B) the decrease in the amplitude of the evoked CAP of the rat sciatic nerve after the incubation of the nerve in 3.4 mg/mL of 3-pentanone (n=4). (C) The nerve was washed in normal saline and the nerve CAP was monitored for 24 h.

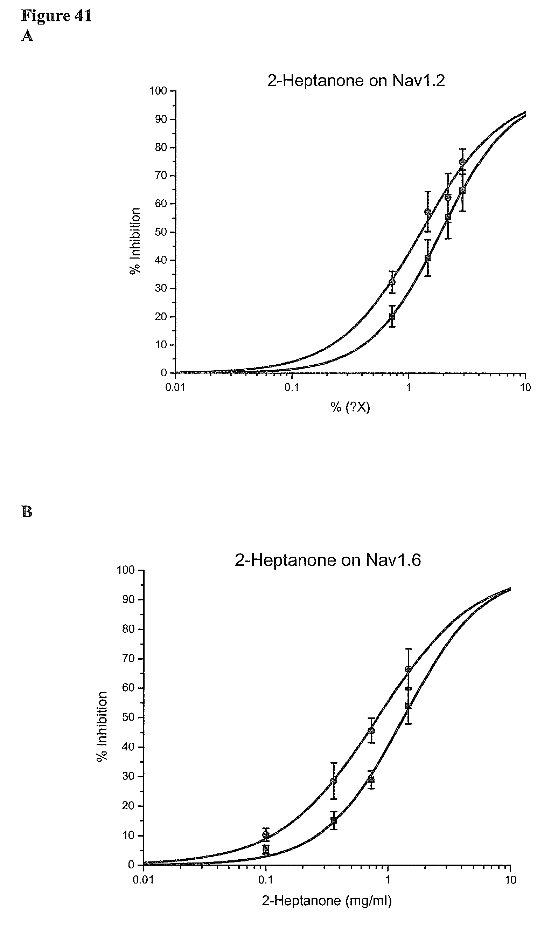

Figure 41 shows the effect of 2-heptanone on Nav1.2 **(A)** and Nav1.6 **(B)** sodium channels. Data represent tonic (filled square) and phasic (filled circle) effects. Solid curves represent when data are fitted with a logistic equation with minimum and maximum effects fixed at 0 and 100 (n=4). **A.** For Nav1.2, the estimated IC₅₀ = 1.89 ± 0.01 mg/ml (mean ± SE) for tonic and 1.28 ± 0.09 mg/ml (mean ± SE) for phasic effect. The Hill coefficient constants are 1.44 ± 0.02 (mean ± SE) for tonic and 1.24 ± 0.17 (mean ± SE) for phasic effect. **B.** For Nav1.6, the estimated IC₅₀ = 1.34 ± 0.08 mg/ml (mean ± SE) for tonic and 0.82 ± 0.03 mg/ml (mean ± SE) for phasic effect. The Hill coefficient constants are 1.35 ± 0.12 (mean ± SE) for tonic and 1.10 ± 0.06 (mean ± SE) for phasic effect.
Figure 42 shows the effects of lidocaine on Nav1.2 and Nav1.6 sodium channels. Curves represent when data are fitted with a logistic equation with minimum and maximum effects fixed at 0 and 100 (n=4). **A.** For Nav1.2, the estimated IC₅₀ = 0.27 ± 0.01 mg/ml (0.99 mM) for tonic effect. **B.** For Nav1.2, the estimated IC₅₀ = 0.10 ± 0.01 mg/ml (0.40 mM) for phasic effect. **C.** For Nav1.6, the estimated IC₅₀ = 0.29 ± 0.01 mg/ml (1.1 mM) for tonic effect. **D.** For Nav1.6, the estimated IC₅₀ = 0.07 ± 0.01 mg/ml (0.26 mM) for phasic effect.

### DETAILED DESCRIPTION

2-heptanone is a substance produced by the mandibular glands of adult worker honey bees, *Apis mellifera* and *Apis cerana* (Vallet et al., J. Insect Physiol. 37(11):789-804 (1991); Sakamoto et al., Journal of Apiculture Research 29(4):199-205 (1990)), older than 8-10 days. The opening of the mandibular gland is inside the buccal cavity (mouth) of the honey bee at the base of the mandibles. 2-heptanone is produced continuously and is universally distributed throughout the honey bee colony and in the wax. It has been suggested that 2-heptanone acts as an alarm pheromone, having a repellant effect and deterring potential invaders [see e.g. Nature 206:530 (1965)]. Furthermore, 2-heptanone has been proposed for use as a chemoattractant in the control of parasitic *Varroa* mites in honey bee colonies (see US 2005/0090560). It is also believed that one of the functions of 2-heptanone in the honey bee hive is that of the principal universal solvent used by the bees to manufacture honey bees wax comb and propolis (bee glue used to suspend wax combs and plug holes). The honey bees secrete 2-heptanone while they use their mandibles to masticate (chew) the tiny wax flakes produced by their abdominal wax glands. The wax flakes are formed into uniformly thin wax sheets that are used to build the solid hexagonal wax walls of honey comb cells.

The present invention is based in part on the surprising finding that heptanone (e.g. 2-, 3- or 4-heptanone) has a powerful and effective local anaesthetic action on mammalian nerves. The effects of 2-heptanone are similar to that of lidocaine, indicating that 2-heptanone acts via inhibition of voltage-gated sodium channels. Accordingly, the present invention provides the use of heptanone (e.g. 2-, 3- or 4-heptanone) as a local anaesthetic.

2-heptanone has the molecular formula CH₃(CH₂)₄COCH₃, and is also known as methyl amyl ketone and methyl pentyl ketone. It is a volatile liquid at room temperature (d₄¹⁵ 0.8197; b.p.₇₆₀ 151.5°C). It is soluble in alcohol or ether, soluble in water (4.3 mg/mL) and available commercially. The isomers 3-heptanone (CH₃(CH₂)₃COCH₂CH₃) and 4-heptanone (CH₃(CH₂)₂CO(CH₂)₂CH₃) have similar physical properties. In a preferred embodiment, 2-heptanone is used.

In embodiments of the present invention, 2-heptanone (or 3-heptanone or 4-heptanone) is used as a local anaesthetic. By "local anaesthetic" it is meant that the compound is used to induce insensitivity of one or more specific regions of the body to pain, typically whilst the subject remains conscious. Thus the local anaesthetic use according to the present invention differs from general anaesthetic use, in which a compound is used to induce a generalized lack of sensation throughout the whole body and/or lack of consciousness.

Typically the compound is used as a local anaesthetic, and has a very similar action to lidocaine. Lidocaine is a commonly used local anaesthetic with a well-characterised inhibitory effect on voltage-gated sodium channels. The examples below demonstrate that 2-heptanone also shows a dose-dependent inhibition of voltage-gated sodium channels. Thus in some embodiments, the compound may be used to inhibit voltage-gated sodium channels. In some embodiments, the effect of the compound on voltage-gated sodium channels may occur at a higher concentration than that of lidocaine.

The present invention also provides a pharmaceutical composition comprising heptanone (e.g. 2-, 3- or 4-heptanone) for use as a local anaesthetic. Such a composition may comprise a therapeutically effective amount of heptanone (e.g. 2-, 3-or 4-heptanone), and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered.

Such pharmaceutical carriers can be sterile liquids, such as water and oils, including those of petroleum, vegetable or synthetic origin, such as peanut oil, soybean oil, mineral oil, sesame oil and the like. Water is a preferred carrier when the pharmaceutical composition is administered by injection. Saline solutions and aqueous dextrose and glycerol solutions can also be employed as liquid carriers, particularly for injectable solutions.

Suitable pharmaceutical excipients include starch, glucose, lactose, sucrose, gelatin, malt, rice, flour, chalk, silica gel, sodium stearate, glycerol monostearate, talc, sodium chloride, dried skimmed milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the compound, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for injection to human beings. Typically, compositions suitable for administration by injection are solutions in sterile isotonic aqueous buffer. Where necessary, the composition may also include a solubilizing agent. Generally, the ingredients are supplied either separately or mixed together in unit dosage form, for example, as a water free concentrate in a hermetically sealed container such as an ampoule or sachette indicating the quantity of active agent. Where the composition is administered by injection, an ampoule of sterile water for injection or saline can be provided so that the ingredients may be mixed prior to administration.

The compound of the invention can be formulated as neutral or salt forms. Pharmaceutically acceptable salts include those formed with cations such as those derived from sodium, potassium, ammonium, calcium, ferric hydroxides, isopropylamine, triethylamine, 2-ethylamino ethanol, histidine, procaine, etc.

The amount of the compound of the invention which will be effective as a local anaesthetic can be determined by standard clinical techniques. In addition, *in vitro* assays may optionally be employed to help identify optimal dosage ranges. The precise dose to be employed in the formulation will also depend on the route of administration, and the duration and depth of local anaesthesia required, and should be decided according to the judgment of the practitioner and each patient's circumstances. Effective doses may be extrapolated from dose-response curves derived from *in vitro* or animal model test systems.

In one embodiment, the concentration of heptanone (e.g. 2-, 3- or 4-heptanone) in the pharmaceutical composition is at least 1 mM, at least 5 mM or at least 10 mM. Typically the concentration of heptanone (e.g. 2-, 3- or 4-heptanone) is less than 38 mM, 35 mM or 30 mM. Thus preferred concentration ranges of heptanone (e.g. 2-, 3-or 4-heptanone) in the composition are e.g. 1 to 38 mM, 5 to 35 mM, or 10 to 30 mM.

The invention also provides a pharmaceutical pack or kit comprising one or more containers filled with one or more of the ingredients of the pharmaceutical compositions for use as a local anaesthetic as described herein. Optionally associated with such container (s) can be a notice in the form prescribed by a governmental agency regulating the manufacture, use or sale of pharmaceuticals or biological products, which notice reflects approval by the agency of manufacture, use or sale for human administration.

Methods of administration of the composition include but are not limited to intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g. oral mucosa, rectal and intestinal mucosa, etc.) and may be administered together with other biologically active agents.

Typically the composition is administered such that it exerts a localized effect, i.e. the composition is administered locally rather than systemically. Preferred administration routes are intradermal, intramuscular, or subcutaneous injection, or topical application. Thus in a specific embodiment, the pharmaceutical composition is administered locally to an area in need of anaesthesia, for example by local infusion during surgery, topical application, e.g. in conjunction with a wound dressing after surgery, by injection, by means of a catheter, by means of a suppository, or by means of an implant, said implant being of a porous, non-porous, or gelatinous material, including membranes, such as sialastic membranes, or fibres In one embodiment, the composition may be formulated as a cream, ointment or gel suitable for topical application.

Preferably the composition is administered to a mammal, e.g. a human.

The invention will be further described with reference to the following examples; however, it is to be understood that the invention is not limited to such examples.

### EXAMPLES

### Studies of 2-heptanone Local Anaesthetic Effect

### I. Methodology

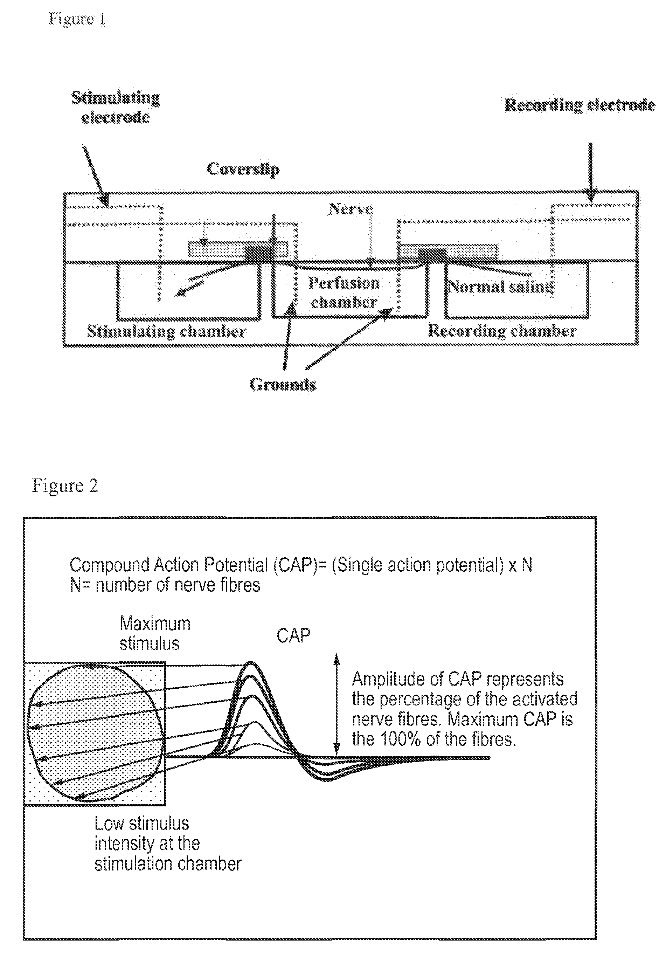

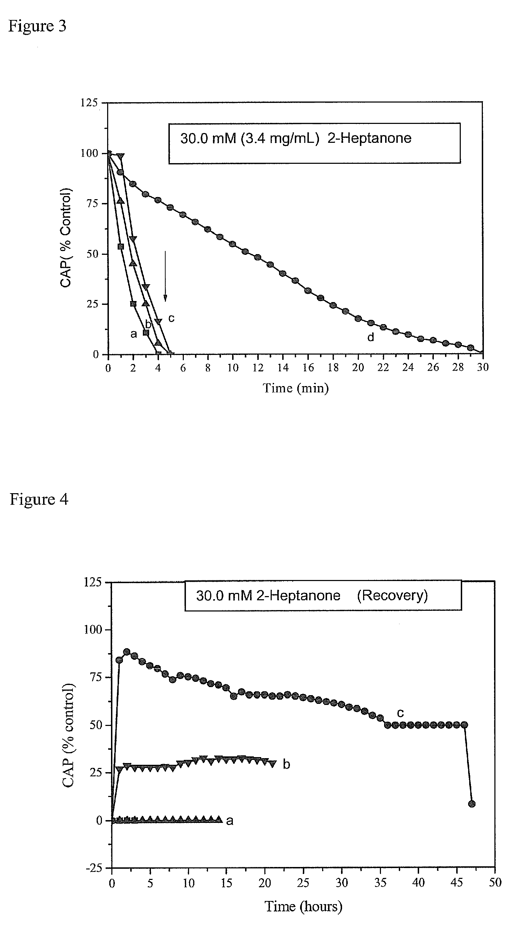

1) The effects of 2-heptanone were tested on the electrophysiological properties of isolated rat sciatic nerve. Male *Wistar* rats (3-4 months old) weighing between 250-300 g were used. Animals were sacrificed through deep anaesthesia using N₂. Other anaesthetics were not used to avoid interaction with the possible local anesthetic action of 2-heptanone.
2) The sciatic nerves were dissected from the spinal cord to the knee, immersed in a standard saline solution (see below) and cleaned under a dissection microscope. In order to achieve direct contact of the nerve fibres and to maximize drug access to all nerve fibres, the epineurium and the perineurium of the nerve were removed.
3) The nerve was mounted across a three-chambered recording bath, made of Plexiglas (Fig.1). Each chamber was filled with oxygenated (O₂, 100%) saline solution of the following composition (in mM): 136 mM NaCl, 11 mM glucose, 4.7mM KCl, 2.4 mM CaCl₂, 1.1 mM MgCl₂, 1 mM NaHCO₃, 10 mM HEPES, pH 7.2). The experiment was performed at a constant temperature of 26 ±1°C.
   The saline solution was used as a vehicle to expose the nerve in 2-heptanone (the 3- and 4-heptanones and other ketones). Each chamber was filled with 12.0 mL saline solution and was oxygenated once, at the beginning of the experiment, and was gently stirred throughout the experiment (for over 30.0 h).
4) In the first chamber (see the recording chamber in Fig. 1) was placed the distal part of the sciatic nerve and the active electrode of an A.C. amplifier (Neurolog, NL822, Digitimer Ltd., UK). In the second, the perfusion chamber (see Fig.1) 70% of the 10 cm long nerve and the grounds of the recording and stimulating electrodes were immersed. In the third, the stimulating chamber (Fig. 1) the proximal part of the nerve and the active stimulating electrode connected to a constant voltage stimulator (Digitimer, DS9A England, UK) were bathed. The dimensions of each chamber were 24 mm length, 36 mm width and 10 mm depth. Each of the three chambers was filled once with 12 ml oxygenated saline to cover the nerve. The saline in the perfusion chamber was continuously stirred with a small magnet.
5) 2-heptanone was added only in the saline of the middle chamber, the perfusion chamber (see perfusion, in Fig.1). 2-heptanone was first diluted into DMSO, in the initial experiments, and then added in to the perfusion chamber. The concentration of DMSO used to dilute 2-heptanone had no effect on the sciatic nerve preparation. The proximal and distal ends of the nerve remained in the saline of the recording and stimulating chambers at all times, and thus were never exposed to the pharmacological agents used. To eliminate effects of DMSO on the local anesthetic action, in most experiments 2-heptanone was diluted in the normal saline in the perfusion chamber (Fig.1 see perfusion chamber) and the concentrations applied to the nerve were 10 mM (1.13 mg/mL), 12.5 mM (1.41 mg/mL), 20 mM (2.26 mg/mL) and 30 mM (3.4 mg/mL). Only when high concentrations were applied, 88.2 mM, the solvent DMSO was used. The action of 2-heptanone on the rat sciatic nerve was compared to the action of lidocaine, a standard local anaesthetic extensively used, and to the action of linalool, an essential oil produced by plants. Lidocaine was tested at the concentrations of 6.0 mM (1.43 mg/mL), 10 mM and 14.5 (or 3.4 mg/mL).
6) In order to monitor vitality of the sciatic nerve fibres in the recording chamber, their evoked compound action potential (CAP) was recorded using standard electrophysiological methods (Fig.2). The propagated monophasic CAPs were measured as the potential difference between the recording and the perfusion chamber. The CAP reflects the summation of the action potentials of the stimulated sciatic nerve fibres, and in the case of supramaximal stimulation, the summation of all of the sciatic nerve fibres (Fig.2 see CAP). The CAP was amplified, digitized, recorded, and analyzed on a computer (Figure 2). The % amplitude of the CAP compare to control was plotted to produce the time-response curves. They represent the inactivation on nerve fibres in the presence of 2-heptanone (examples Figure 3 a,b,c,d).

### II. Results

The experiments were performed under two conditions: 1) the saline in the perfusion chamber was stagnant throughout the experiment and 2) the saline was continuously stirred by a small magnet in the perfusion chamber. The condition is mentioned for each experiment (see below). 3) 2-Heptanone was diluted in the saline at concentrations below 38.00 mM or 4.3 mg/mL. This is the solubility of 2-heptanone in water.

### Example 1

### 1. The effect of 30 mM (3.4 mg/mL) 2-Heptanone on the sciatic nerve of the rat (stagnant saline)

The amplitude of the evoked compound action potential (CAP) of the rat sciatic nerve decreased after the incubation of the nerve at 30.0 mM 2-heptanone **(****Figure 3****).** Three of the nerves (curves a, b, c) incubated in 30 mM 2-heptanone had a similar response, with an *IT₀ of 4-5 min (240-300 s). The 4^{th} experiment (d) has an IT₀ of 28 min. * IT₀: is the time required to eliminate, bring to zero, the evoked nerve Compound Action Potential (CAP) (see Methodology above).

### Example 2

### 2. The recovery after exposure to 30 mM 2-heptanone

In the previous experiment (see Figure 3), after the CAP was eliminated (i.e reached a values near 0), the nerve in the perfusion chamber (which had been exposed to 30.0 mM 2-Heptanone), was washed and the solution in the chamber was replaced with normal saline.

As shown in Figure 4, recovery of the evoked CAP varied between different nerves (a, b and c).

### Example 3

### 3. A. The effects of 15 mM (1.7 mg/mL) 2-heptanone (stagnant saline)

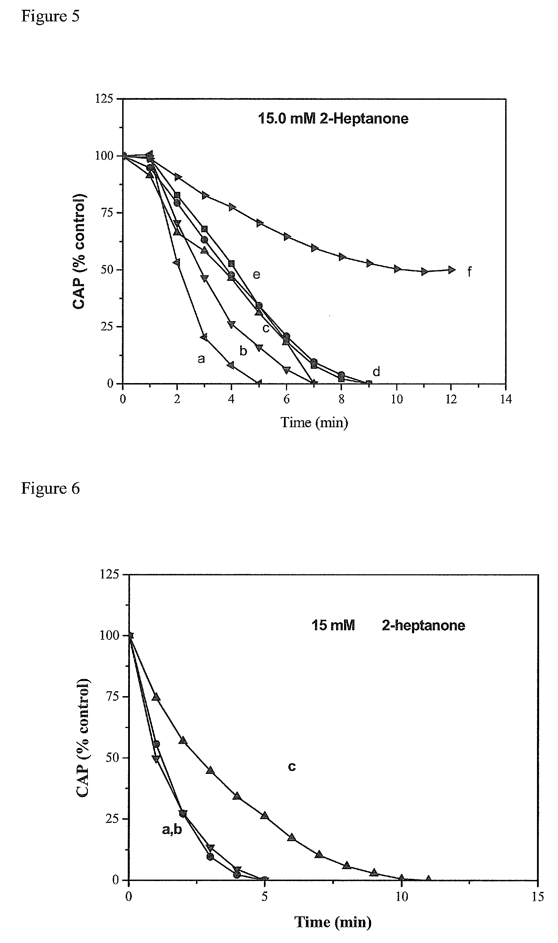

The amplitude of the evoked CAP of the rat sciatic nerve decreased following incubation in 15.0 mM (1.7 mg/mL) 2-heptanone in stagnant saline (see Figure 5). In the presence of 15.0 mM 2-heptanone, the CAP of 5 nerves was eliminated within 5-9 min (curves a-e). The IT₀ is 5-9 min (n=5).

### 3. B The effects of 15 mM 2-heptanone (stirred saline)

The experiments with 15 mM (1.7 mg/mL) 2-heptanone on another 3 nerves are shown in Figure 6 (see below). In this case the saline with the 2-heptanone was stirred continuously.

The CAP of 2 nerves was eliminated within 5 min (curve a,b). The IT₀ is 5 min (300 s). For the 3^{rd} nerve the IT₀ was 10 min. The comparison with Figure 5 (15 mM stagnant) shows that for the specific concentration, 15 mM, there is no significant difference between the nerves exposed in stagnant saline and those where the saline was stirred continuously.

### Example 4

### 4.A The recovery after exposure to 15 mM 2-heptanone (in stagnant saline)

The sciatic nerve of the Example 3A, which had been exposed to 15.0 mM 2-heptanone (see Fig.5), was washed and the perfusion solution replaced with normal saline.

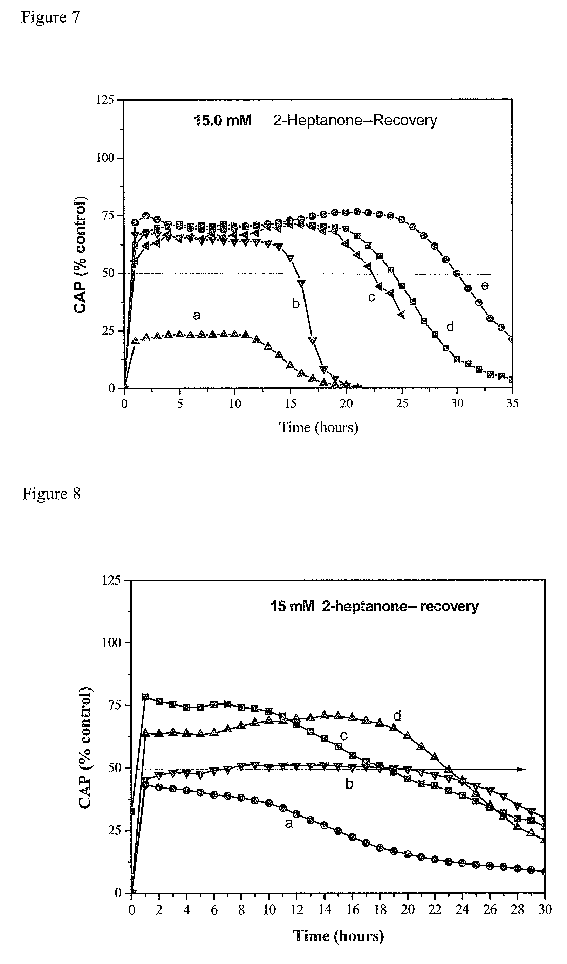

As shown in Figure 7, there is an instant recovery of the evoked CAP. One nerve showed less recovery (curve a, near 25% of the control nerve). However, there was an almost full recovery of the other four nerves (b-e), between 60 to 75 % of the control.

### 4.B The recovery after exposure to 15 mM 2-heptanone (in stirred saline)

The sciatic nerve of the Example 3B, which had been exposed to 15.0 mM 2-heptanone and stirred continuously (see Fig.6), was washed and the perfusion solution replaced with normal saline.

Figure 8 shows the recovery of the CAP of nerves after exposure to 15mM 2-heptanone where in the saline was stirred continuously.

### Example 5

### 5. A The effects of 10 mM (1.13 mg/mL) 2-heptanone (stagnant saline)

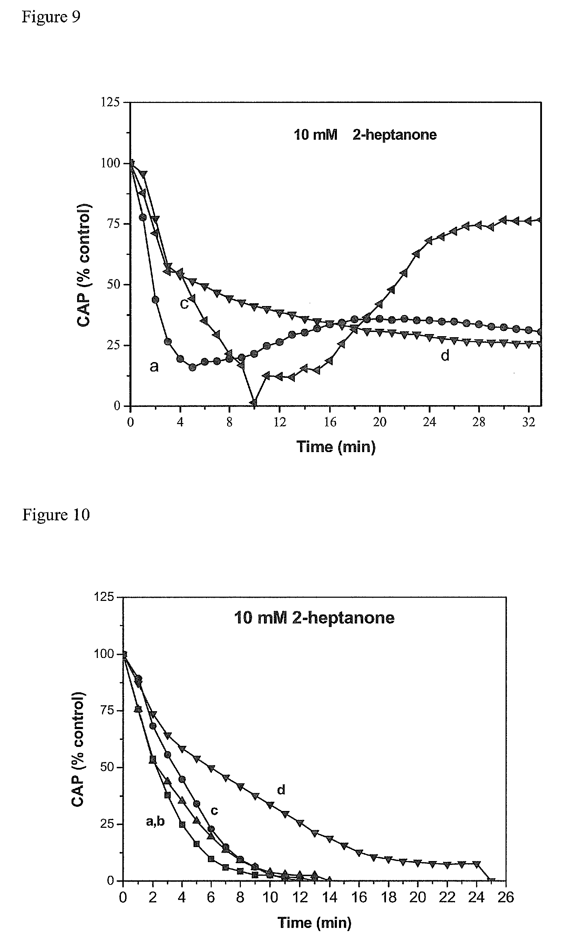

As shown in Figure 9, the CAP amplitude of the rat sciatic nerve decreased following incubation in the presence of 10.0 mM 2-heptanone. However, the inhibitory effect at this concentration is less marked than at 15 or 30 mM 2-heptanone, indicating that 10 mM is nearer to the threshold concentration for a local anaesthetic effect.

### 5. B The effects of 10 mM 2-heptanone on the sciatic nerve of the rat (stirred saline)

The same experiments were repeated, with 10 mM 2-heptanone, but in stirred continuously saline. Under these conditions the action of 2-heptanone was significantly improved (see Figure 10). For the 3 nerves the IT₀ was between 11 to 14 min (curves a,b,c) and in a fourth nerve an IT₀=26 min was observed (curve d).

2-Heptanone appears to perform better as a local anesthetic in stirred saline, therefore for the rest of the experiments the saline with the main part of the nerve was continuously stirred.

### Example 6

### 6.A. The recovery after exposure to 10 mM 2-heptanone (stagnant saline)

A) The sciatic nerves from the previous experiments exposed to 10.0 mM 2-heptanone (see Figure 9), were washed and the saline with 2-heptanone was replaced with normal saline (the saline was stagnant).

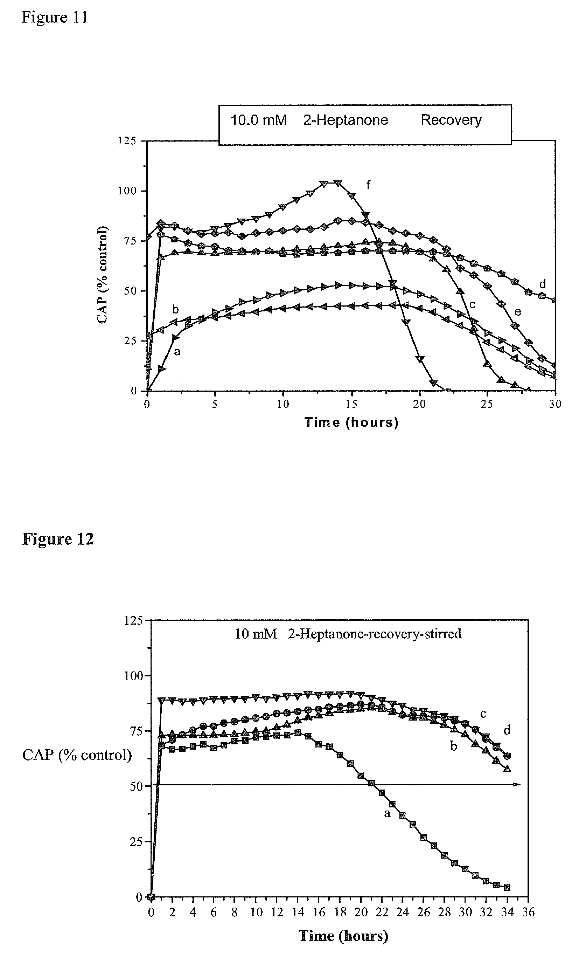

As shown in Figure 11, there is a 25-50% recovery of the CAP in two nerves (curves a,b) and a 65-80% recovery in 4 nerves (curves c,d,e,f).

### 6. B. The recovery after exposure to 10 mM 2-heptanone (stirred saline)

In the experiments described in Figure 10, after the CAP was eliminated the perfusion solution was replaced with normal saline (stirred). The results are shown in Figure 12.

There is a 75-90 % recovery in three nerves (curves a, b and c) and 90 % recovery in one nerve (curve d). The high vitality of the nerves during recovery is shown by the fact that the CAP remained above 50% of the control value (see horizontal line) for at least 20 hours for all nerves, and for at least 32 hours for 2 nerves.

Also in the recovery, 2-heptanone appears to perform better as a local anesthetic in stirred saline, therefore for the rest of the experiments the saline with the main part of the nerve was continuously stirred.

### Comparative Example

### C. The effects of 10 mM (or 2.35 mg/mL) lidocaine on the sciatic nerve of the rat (stirred)

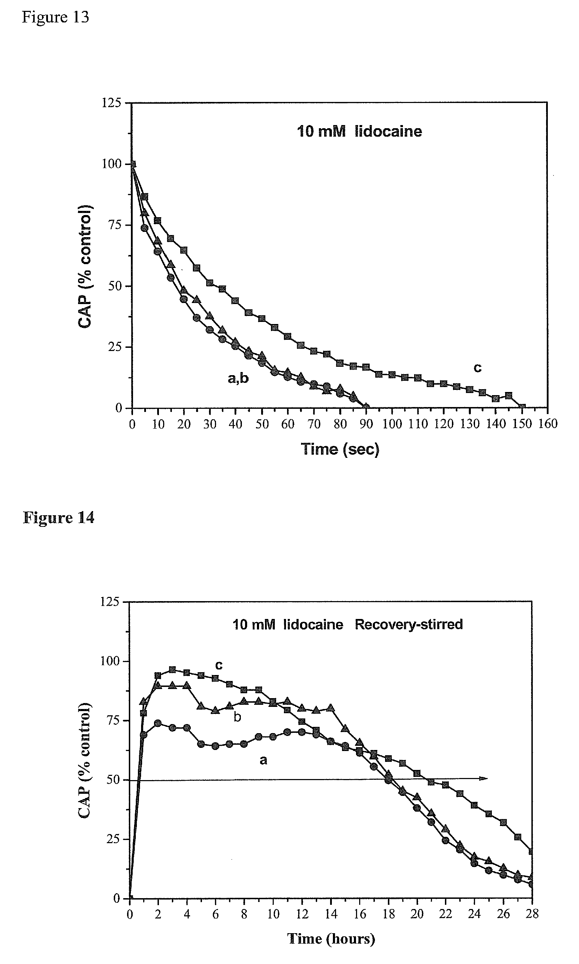

In the presence of 10.0 mM lidocaine, the CAP of the 3 nerves examined was eliminated within 1 to 3 min. The IT₀ was 90 sec (or 1.5 min) for the two nerves (curves a-c Figure 13). The IT₀ for the 3rd nerve was 150 s or 2.5 min.

### D. The recovery after exposure to 10 mM lidocaine (stirred)

In the experiments described above (Figure 13), as soon as the CAP was eliminated the nerves were washed and incubated in normal saline (stirred). Figure 14 shows the recovery of the CAP amplitude after the washout of 10.0 mM lidocaine. There was an instant recovery of the CAP. All three nerves recovered to about 70 to 80 % of the initial value (the CAP before the application of lidocaine).

However the vitality of the sciatic nerve after the removal of lidocaine, as represented by the time the CAP remains above the 50% of the control value (see horizontal line) during recovery), was reduced compared to 2-heptanone-treated nerves. In those particular experiments, in the lidocaine-treated sciatic nerve, the CAP was at or below 50% of control value within 20 hours (see Figure 14).

### Conclusions

The results shown above demonstrate that 2-heptanone has a concentration-dependent local anaesthetic action at concentrations 10 to 30 mM (see Fig.s 3, 5, 6, 8 and 9). At a concentration of 10 mM 2-heptanone has a good local anaesthetic action, with an IT₀ of 10 to 12 min when the saline is stirred (Fig. 10). In identical experimental conditions lidocaine has an IT₀ of 1.5 to 2.5 min (Fig 13).

The results so far have shown that although the action of lidocaine appears to be faster, there is a significant difference in the quality of the recovery of the CAP. For 10 mM lidocaine, during recovery the CAP remains above its 50% value for less than 20 h (see Fig. 14). In contrast for 10 mM 2-heptanone, during the recovery phase the nerve remains above its 50% value for over 25 hours. This is an indication that for the concentration of 10 mM (or 1.13 mg/mL) 2-heptanone appears to be less neurotoxic compared to the classical local anaesthetic lidocaine. The effects at other concentrations were studied further below.

### Example 7

### The effects of 2-heptanone on ion channels expressed in mammalian cells

In this example, the *in vitro* effects of 2-heptanone on the following ion channels were evaluated at room temperature using the PatchXpress 7000A (Molecular Devices), an automatic parallel patch clamp system:
1. Cloned hNav1.2 sodium channels (SCN2A gene, expressed in CHO cells).
2. Cloned hNav1.6 sodium channels (SCN8A gene, expressed in CHO cells).

The effects of 2-heptanone were evaluated at 0.73, 1.46, 2.19, and 2.92 mg/ml with each concentration tested in two cells (n = 2). The duration of exposure to each concentration of 2-heptanone was 5 minutes. A summary of the results is shown in Table 1. Tables 2 to 5 show the individual results from each concentration followed by individual concentration-response curves. The positive controls (Table 6) confirm the sensitivity of the test systems to inhibition.

**Table 1**

| **Channel** | | **2-Heptanone IC₅₀ (mg/ml)** | **2-Heptanone IC₅₀ (mM)** |
|---|---|---|---|
| Nav1.2 | Tonic | 1.380 | 12.11 |
| | Phasic | 0.864 | 7.58 |
| Nav1.6 | Tonic | 0.806 | 7.07 |
| | Phasic | < 0.73 | 6.40 |

**Table 2 Nav1.2 tonic inhibition by 2-heptanone**

| **IC50 (mg/ml)** | **Conc. (mg/ml)** | **Mean % Nav1.2 Tonic Inhibition** | **Standard Deviation** | **Standard Error** | **n** | **Individual Data Points (% Inhibition)** |
|---|---|---|---|---|---|---|
| 1.380 | 0.73 | 29.7 | 2.6 | 1.8 | 2 | 31.5 |
| | | | | | | 27. |
| | 1.46 | 47.6 | 2.5 | 1.8 | 2 | 49.3 |
| | | | | | | 45.8 |
| | 2.19 | 65.1 | 12.0 | 8.5 | 2 | 56.7 |
| | | | | | | 73. |
| | 2.92 | 83.9 | 7.3 | 5.2 | 2 | 78.7 |
| | | | | | | 89. |

**Table 3 Nav1.2 phasic inhibition by 2-heptanone**

| **IC50 (mg/ml)** | **Conc. mg/ml)** | **Mean % Nav1.2 Phasic Inhibition** | **Standard Deviation** | **Standard Error** | **n** | **Individual Data Points (% Inhibition)** |
|---|---|---|---|---|---|---|
| 0.864 | 0.73 | 44.4 | 0.2 | 0.2 | 2 | 44.3 |
| | | | | | | 44.6 |
| | 1.46 | 66.3 | 2.3 | 1.6 | 2 | 67.9 |
| | | | | | | 64.7 |
| | 2.19 | 90.0 | 3.4 | 2.4 | 2 | 87.6 |
| | | | | | | 92.4 |
| | 2.92 | 99.0 | 1.2 | 0.8 | 2 | 98.2 |
| | | | | | | 99.8 |

**Table 4 Nav1.6 tonic inhibition by 2-heptanone**

| **IC50 (mg/ml)** | **Conc. (mg/ml)** | **Mean % Nav1.6 Tonic Inhibition** | **Standard Deviation** | **Standard Error** | **n** | **Individual Data Points (% Inhibition)** |
|---|---|---|---|---|---|---|
| 0.806 | 0.73 | 46.8 | 8.3 | 5.9 | 2 | 40.9 |
| | | | | | | 52.6 |
| | 1.46 | 70.6 | 0.3 | 0.2 | 2 | 70.8 |
| | | | | | | 70.4 |
| | 2.19 | 98.3 | 2.5 | 1.8 | 2 | 96.5 |
| | | | | | | 100.1 |
| | 2.92 | 98.0 | 2.7 | 1.9 | 2 | 96.1 |
| | | | | | | 99.9 |

**Table 5 Nav1.6 phasic inhibition by 2-heptanone**

| **IC50 (mg/ml)** | **Conc. (mg/ml)** | **Mean % Inhibition** | **Standard Deviation** | **Standard Error** | **n** | **Individual Data Points (% Inhibition)** |
|---|---|---|---|---|---|---|
| <0.73 | 0.73 | 87.6 | 9.1 | 6.4 | 2 | 94.0 |
| | | | | | | 81.1 |
| | 1.46 | 100.9 | 1.4 | 1.0 | 2 | 101.9 |
| | | | | | | 99.9 |
| | 2.19 | 101.4 | 0.5 | 0.4 | 2 | 101.8 |
| | | | | | | 101.0 |
| | 2.92 | 100.9 | 0.3 | 0.2 | 2 | 101.1 |
| | | | | | | 100.7 |

**Table 6 Positive control (inhibition of Nav1.2 and Nav1.6 by 2 mM lidocaine)**

| **Ion channel** | **Cone. (mM) (mg/mL** | **Mean % Inhibition** | **Standard Deviation** | **Standard Error** | **n** | **Individual Data Points (% Inhibition)** |
|---|---|---|---|---|---|---|
| Nav1.2 | 2 (0.46) | 75.5 | 0.8 | 0.6 | 2 | 76.1 |
| | | | | | | 74.9 |
| Nav1.6 | 2 (0.46) | 61.5 | 3.2 | 2.3 | 2 | 63.8 |
| | | | | | | 59.3 |

### Further examples

### The methodology

### Methodology I. Ex vivo methodology for assessing and comparing the local anesthetic action on the sciatic nerve of the rat

The experiments in the following Examples were performed generally according to the methods set out above, with further details given below.
1) The present method has been used extensively for the *ex vivo* assessment of the action of local anesthetics on the sciatic nerve of rat and frog [see e.g. Geronikaki et al. (2003) Study of local anesthetic activity of some derivatives of 3-amino-benzo-[d]-isothiazole. SAR QSAR Environ Res 14: 5-6. 485-495].
2) All experimental procedures were conducted in accordance with the animal care protocols outlined by Aristotle University of Thessaloniki, Greece and the Veterinary Authorities, and these protocols respect the recommended standard practices for Biological Investigations (license: Protocol Number 144256/864-09/06/2011).
3) Apart from 2-heptanone and lidocaine other local anesthetics (mevipacaine, aticaine, prilocaine) were tested and compared to 2-heptanone. Also other ketones like 3-heptanone, 4-heptanone, 2-pentanone, 3-pentanone, 2-hexanone, 2-octanone and 3-octanone were examined for comparison at the same concentration (3.4 mg/mL).
   From the time response curves, it was possible to estimate the time required for the compounds tested to inhibit the amplitude of the CAP to 50% of its control value, called IT₅₀ (inhibitory time 50%). This is the half-vitality time.
   Also from the time-response curves the IT₀, the time required to eliminate (i.e. bring to zero) the evoked nerve Compound Action Potential (CAP), was estimated.
   From the time-response curves the concentration required to inhibit the CAP to 50% of its control value (IC50), was estimated.
4) After the CAP was completely eliminated by either 2-heptanone or lidocaine, the nerve in the perfusion chamber was washed thoroughly and the nerve was left in normal saline to recover for the rest of the experiment. The vitality of the nerve fibres was monitored for 24 h. This allows the monitoring of the % of recovery and the vitality of the nerve in the presence of the compounds. It was possible to monitor the vitality of the sciatic nerve, the amplitude of the nerve CAP, for more that 24 h, but recordings and the preparation became unstable to produce accurate results during repetition.

Thus the *ex vivo* experiments were performed under specific conditions: 1) the saline in the perfusion chamber was stagnant throughout the experiment, 2) the saline was continuously stirred by a small magnet in the perfusion chamber, 3) 2-heptanone was diluted in saline only. DMSO was used only when the concentration of 10.0 mg/mL (88.2 mM) was tested. The condition is mentioned for each experiment (see below).

### Methodology II. In vivo assessment of the local anaesthetic action on muscles

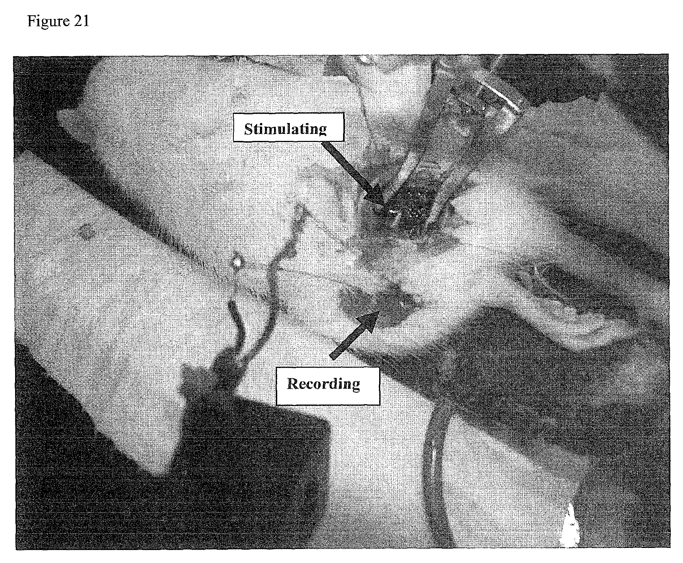

This is a new methodology applying *in vivo* for the assessment and comparison of the action of 2-heptanone and lidocaine. In clinical practice local anaesthetics are also used to eliminate muscular contraction. The effect of intramuscular injected 2-heptanone and lidocaine were also compared using an *in vivo* method. In an anesthetised rat (endoperitoneal chloral hydrate 4%) we inserted two recording electrodes into the proximal region of the gastrocnemius muscle (or biceps) through a small "window" in the skin (see Recording, Fig. 21). We placed a stimulating electrode on the proximal region of the sciatic nerve to evoke muscle CAP (mCAP) every second (see Stimulating, Fig. 21).

The compounds were injected into the middle region of the gastrocnemius muscle or the biceps, using 150 µl saline and Tween80 as a vehicle. After testing a number of serial dilutions, we found that concentration causing a fast decrease of the mCAP. 10.8 mg/kg of lidocaine caused a rapid decrease of the mCAPs. Records were obtained for at least 3 h to estimate the recovery time due to the fact that both lidocaine and 2-heptanone were metabolised. The analysis of the data was made as described above (*ex-vivo* study). The only difference was that instead of the amplitude of the muscle CAP we were measuring the integral of the muscle CAP.

In some cases there was a variation in the experimental data, those performed using intramuscular injection. This could be due: to the size of the muscle, the response to anaesthesia by the animal, the treatment with the Tween 80, the side of the injection of the compound and the even the region of the recordings.

### Example 8

### The local anaesthetic effect of 3.4 mg/mL (30 mM) 2-heptanone on the sciatic nerve of the rat

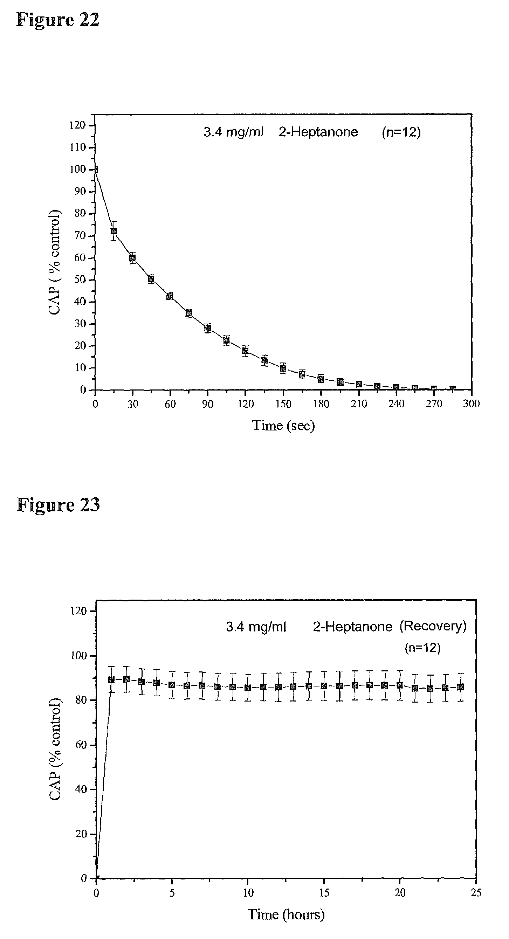

*Inhibition of the CAP:* The amplitude of the evoked nerve compound action potential (CAP) of the rat sciatic nerve decreased during incubation of the nerve at 3.4 mg/mL 2-heptanone. The results of 12 nerves are summarised in Figure 22. The IT₅₀ (see above for terminology) was 39.14 s *(n* =12, SEM: ± 4.84). The IT₀, (the time required to eliminate, i.e. bring to zero, the evoked nerve CAP) was 221.88s (*n*=12, SEM ± 7.32). The results are summarised in Table 7.
*Recovery of the CAP after wash:* In the previous experiment (see Figure 22), after the CAP was eliminated (i.e reached a value near 0), the nerve in the perfusion chamber which had been exposed to 3.4 mg/mL (30.0 mM) 2-Heptanone, was washed and the solution in the chamber was replaced with normal saline. As shown in Figure 23, recovery of the evoked CAP reached values above 89.5 % of the control for all 12 nerves examined (Fig.22).

**Table 7 Summarising the effects of 2-heptanone and lidocaine on the isolated sciatic nerve of the rat (ex-vivo)**

| **Substance** | **Concentration** | **IT₅₀ seconds** | **IT₁₀₀ seconds** | **N** |
|---|---|---|---|---|
| 2-Heptanone | 10 mg/mL (88.2 mM) | 22.3 ±1.1 | 195 ± 12 | 7 |
| | 3.4 mg/mL (30 mM) | 39.14±4.84 | 221.88±7.32 | 12 |
| | 2.28 mg/mL (20 mM) | 108.9±0.22 | 358.8±18.07 | 4 |
| | 1.41 mg/mL (12.5 mM) | 109.7±2.87 | 517.5±25.62 | 4 |
| IC₅₀ | 0.469 mg/mL (SEM ±0.002) | | | |
| Lidocaine | 10.0 mg/mL (42.5 mM) | 12.0 ± 0.95 | 101.3 ± 2.16 | 14 |
| | 3.4 mg/mL (14.5 mM) | 27.11 ± 3.16 | 147.50 ± 9.96 | 12 |
| | 1.41 mg/mL (6.0 mM) | 32.23 ± 3.16 | 320.3 ± 14.96 | 6 |
| IC₅₀ | 0.337 mg/mL (SEM ± 0.001). | | | |

### Example 9

### The local anaesthetic effect of 2.28 mg/mL (20 mM) 2-heptanone

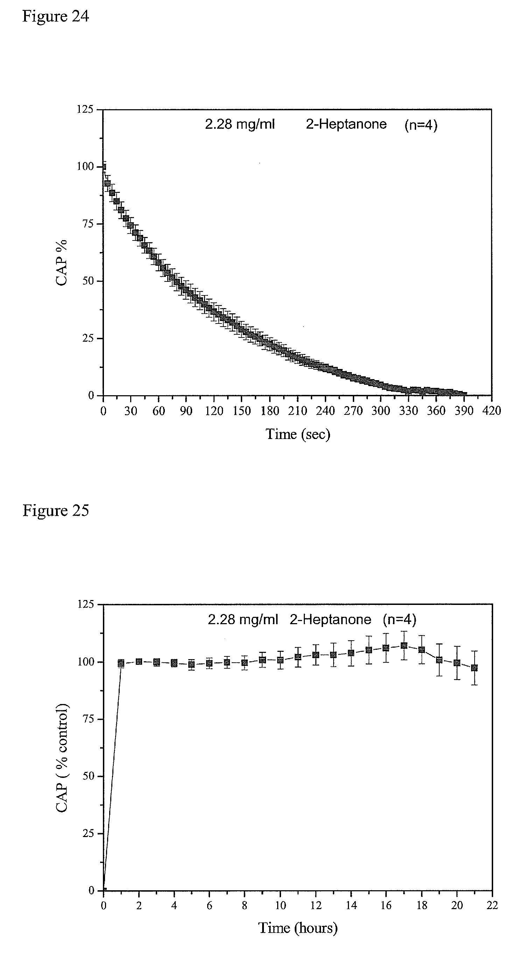

*Inhibition of the CAP:* The amplitude of the evoked CAP of the rat sciatic nerve decreased during incubation in 2.28 mg/mL (20.0 mM) 2-heptanone in saline (see Figure 24). The CAP of 4 nerves was eliminated with an IT₀ of 358.8±18.07 seconds (n=4). The IT₅₀ was 108.9±0.22 seconds (n=4) (see also Table 7).
*Recovery of the CAP after wash:* The recovery of the nerve CAP was near 100% (Figure 25) and remained at this level for 24 h.

### Example 10

### A. The local anesthetic effect of 1.41 mg/mL (12.5 mM) 2-heptanone

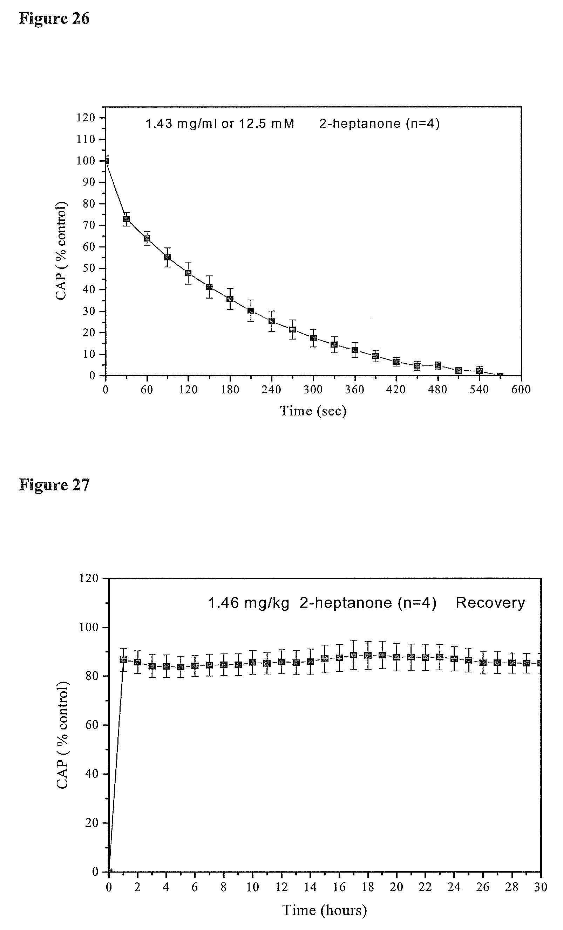

*Inhibition of the CAP:* The amplitude of the evoked nerve CAP of the sciatic nerve decreased after the incubation of the nerve at 1.41 mg/mL 2-heptanone. The results of 4 nerves are summarised in Figure 26. The IT₅₀ was 109.7±2.87 seconds for 2-heptanone *(n* = 4). The IT₀ (the time required to eliminate, i.e. bring to zero, the evoked CAP) was 517.5±25.62 seconds (n=4) (see also Table 7).
*Recovery of the CAP after wash:* The recovery of the nerve CAP was above 87.5% of the control and remained at this level for 24 h (n=4) (Fig. 27).

### B. Comparative example - 2-heptanone vs lidocaine

### The local anesthetic effects of 3.4 mL/mL lidocaine on the sciatic nerve

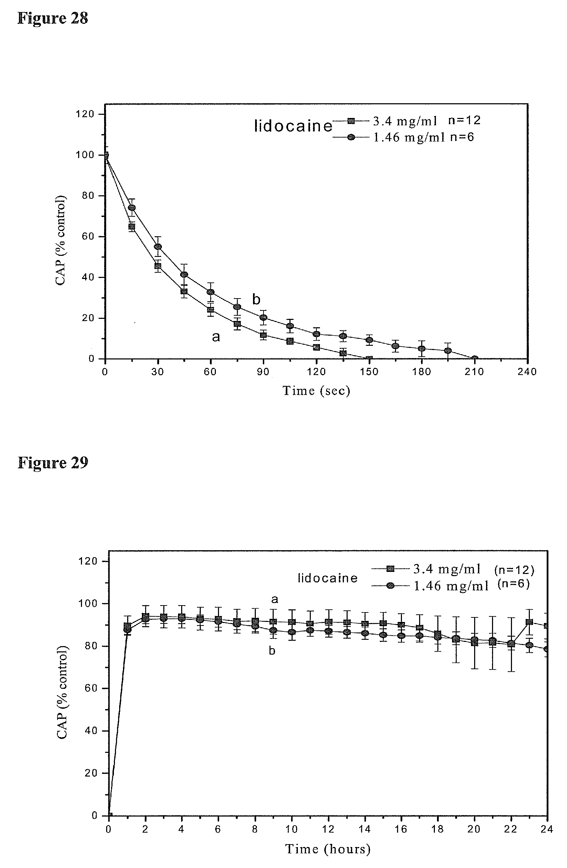

*Inhibition of the CAP:* In the presence of 3.4 mg/mL lidocaine the IT₅₀ was 27.11± 3.16 seconds *(n* = 12) and the IT₀ was 147.50 ± 9.96 seconds (*n* = 12) (from Fig. 28, curve a and Table 7).
*Recovery of the CAP:* After washout of the nerve with normal saline the recovery of the CAP was 95.5% of the control (Fig. 29, curve a) and remained at this level for 24 h.

### The local anesthetic effects of 1.41 mg/mL (6.0 mM) lidocaine

*The inhibition of the CAP:* In the presence of 1.41 mg/mL lidocaine the IT₅₀ was 32.23± 3.16 seconds *(n* = 6) and the IT₀ was 320 ± 14.96 seconds *(n* = 6) (see Fig. 28, curve b and Table 7).
*Recovery of the CAP:* After the nerve was washed with normal saline, the recovery of the CAP was 95% (Fig. 29, curve b) and remained at this level for 24 h.

### Comparing the local anesthetic effects of 2-heptanone and other local anaesthetics Example 11

### A. Comparing 3.4 mg/mL of 2-heptanone and lidocaine

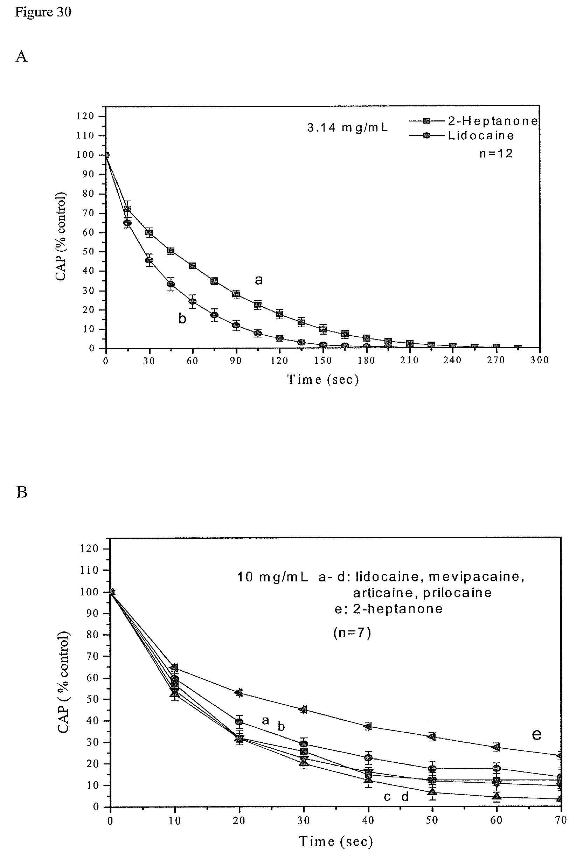

*Inhibition of the CAP (2-heptanone vs lidocaine):* The amplitude of the CAP of the rat sciatic nerve decreased after the incubation of the nerve at 3.4 mg/mL 2-heptanone and lidocaine as follows:
The IT₅₀ was 39.14± 4.84 s *(n* = 12) for 2-heptanone and 27.11 ± 3.16 seconds (*n* = 12) for lidocaine (from Fig. 30A, curves a and b).
The IT₀ was 221.88 ± 7.32 seconds (*n*=12) for 2-heptanone and 147.50 ± 9.96 seconds for lidocaine (from Fig. 30A, curves a and b).

When higher and lower concentrations were tested it was possible to estimate the IC₅₀ for the two compounds which for 2-heptanone was mean 0.469 ±0.002) and lidocaine mean 0.337±0.001 mg/mL. The IC₅₀ values were estimated using the program GraphPad Prism 5.

The values of IT₅₀, IT₀ and IC₅₀ for 2-heptanone and lidocaine and the number of experiments (n) are summarised in Table 7.

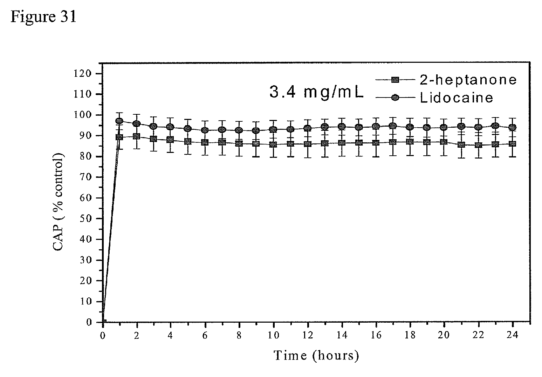

*Recovery of the CAP:* for both cases, 2-heptanone and lidocaine, after wash with normal saline the recovery of the CAP was 89.5 and 95.5% correspondingly and remained at this level for more than 24 h (Figure 31). In the recovery time-response curves there was no significant difference between the values of 2-heptanone and lidocaine (P>0.05).

### B. Comparing the local anesthetic effect of 10 mg/mL 2-heptanone and 4 (four) different local anaesthetics

*Inhibition of the CAP by 10 mg*/*mL of 2-heptanone and 4 other local anaesthetics:* The amplitude of the CAP of the rat sciatic nerve decreased during incubation in 10 mg/mL 2-heptanone and 4 other local anaesthetics, as shown in Figure 30B.

The IT₅₀ for the four (lidocaine, mevipacaine, aticaine, prilocaine, curves a, b, c, d) was between 12 to 15 seconds while for 2-heptanone (curve e) was between 22-23 (mean 22.3 ±1.1 seconds, n=7, see also Table 7).

Within the time limit of 70 seconds of treatment, and with minimum and maximum effects fixed at 0 and 100 (Fig. 30 B), the 4 local anesthetics decrease the CAP to 5-17 % while 2-heptanone to 24% (n=7) (values estimated from Fig. 30 B).

### Example 12

### Comparing the effects of 2-heptanone and lidocaine on the muscles of the rat: an in vivo study

*Lidocaine:* after testing a number of serial dilutions, we found that *in-vivo* intramuscular injection of 10.8 mg/kg of lidocaine caused a rapid decrease of the integral of mCAPs (Fig. 32A curve a), with an IT₅₀ of 19.30 ± 1.92 seconds (*n* = 9). Over the subsequent 1920 s (31 min), the mCAPs ultimately plateau at 20.83 ± 2.32% (n= 9) (Data not shown).
*2-Heptanone:* after testing of a number of dilutions we found that *in-vivo* intramuscular injection of 7.2 mg/kg 2-heptanone, diluted in Tween 80 + 0.9% NaCl, caused also a rapid decrease of the integral of the mCAPs (Fig. 32A curve b) with an IT₅₀ of 25.78 s *(n* = 12, SEM: ± 2.87). A plateau of 16.19% (*n* = 12, SEM: ± 2.78%) over the subsequent 1920 s (Fig. 32A curve b) was created. 2-heptanone was diluted in 0.84 µL Tween 80 and 115.48 µL of 0.9 % NaCl, before the injection.
*Tween 80:* The quantity of 0.84 µL Tween 80 and 119.16 µL of 0.9 % NaCL was injected as control (curve c, in Fig. 32A). From the 9 experiments injecting this mixtured only, as control in the gastrocnemius muscle, six (6) experiments showed a small, 10-12% decrease of the mCAP which almost immediately recovered to the control values. These are the experiments shown in Figure 32A (curve c).

In one (1) experiment there was a 50 % decrease in the mCAP which lasted as long as the experiment. In two (2) experiments there was a decrease near 25% which remained at this level through out the experiment. These experiments were excluded from the mean shown in Figure 32A (curve c).

The action of 2-heptanone was dose-dependent; at 10.8 mg/kg (3 mg/ml) the IT₅₀ was 11.16 s *(n* = 12, SEM: ± 0.85) and the plateau was 5.36 ± 1.26%, but there was no recovery after 3h (data not shown).

*2-heptanone on the biceps muscle:* The same experiments using 7.2 mg/kg 2-heptanone were performed *in-vivo* on the biceps muscle. After intramuscular injection of 7.2 mg/kg 2-heptanone diluted in 0.563 µL Tween 80 plus 76.98 µL 0.9 %NaCl, there was a rapid decrease of the mCAPs (Fig. 32B curve a) with an IT₅₀ of 9.13± 0.73 s *(n* = 6) and a plateau at 33.96±4.24 % *(n* = 6), over the subsequent 1920 s (Fig. 32B curve a). The quantity of 0.56 µL Tween 80 and 79.44 µL of NaCl 0.9% was injected as control (curve b). Eight experiments were used for the testing of the mixture alone as a control. In all cases there was a small decrease 12-17% of the mCAP, but a fast recovery to a level of 90-100%.

We also tested higher and lower concentrations, which either permanently inhibited the mCAP or were not effective at all (quantities < 7.2 mg/kg). The IT₅₀ values of 7.2 mg/kg 2-heptanone and 10.8 mg/Kg lidocaine on the gastrocnemius muscle were similar (*P* > 0.05), an indication that 2-heptanone was 1.5 times more effective than lidocaine when tested on a muscle.

***Recovery of the mCAP in vivo:*** The mCAP of the gastrocnemius muscle injected with 10.8 mg/kg lidocaine recovered to 70.3± 8.67% (*n* = 9), of the initial values after 3h of continuous recording. The elimination half-life of lidocaine following an intravenous bolus injection is typically 1.5 hours when tested in other mammals and humans.

The mCAP from the gastrocnemius muscle injected with 7.2 mg/kg 2-heptanone recovered to 65.4± 7.78 % *(n* = 8), after 3h of continuous recording, an indication that 2-heptanone was also metabolized equally well to lidocaine in mammals.

### Comparing 2-heptanone with the other ketones

### Example 13

### The local anaesthetic effect of 3.4 mg/ml of 4-heptanone on the sciatic nerve

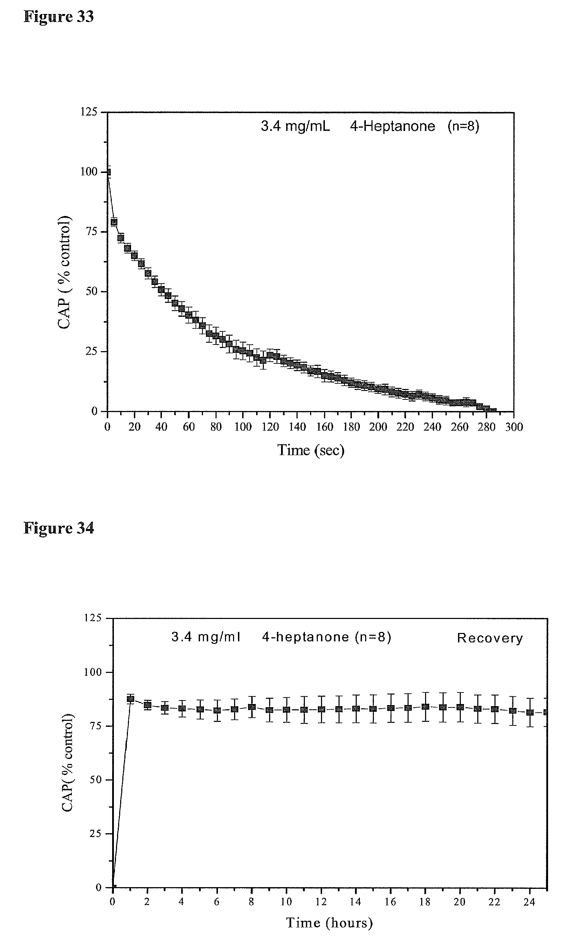

*Inhibition of the CAP:* The amplitude of the evoked CAP of the rat sciatic nerve decreased during incubation in 3.4 mg/mL 4-heptanone in saline (see Figure 33). The CAP of 8 nerves was eliminated within 283.57±16.89 seconds (IT₀). The IT₅₀ was 45.24±0.44 (n=8) (see Table 8).
*Recovery of the CAP after wash :* The recovery was 82% within 1 h and remained at this level for at least 24 h (Figure 34).

**Table 8 Comparing the local anesthetic properties of 2-heptanone with the other ketones**

| **Substance 3.4 mg/mL** | **IT₅₀** | **IT₁₀₀** | **N** |
|---|---|---|---|
| 2-Heptanone | 39.14±4.84 | 221.88± 7.32* | 12 |
| 3-Heptanone | 40.82±1.67 | 258.00±11.47 | 6 |
| 4-Heptanone | 45.24±0.44 | 283.57±16.89 | 8 |
| 2-Hexanone | 74.42±0.79 | 401.00±33.20 | 7 |
| 2-Octanone | 81.45±1.58 | 555.00±75.00 | 4 |
| 3-Octanone | 117.10±0.53 | 722.50±95.66 | 6 |
| 2-Pentanone | 390.60±0.10 | No measurable | 6 |
| 3-Pentanone | 230.30±1.29 | 765.00±86.60 | 4 |

| | | | |
|---|---|---|---|
| * ITo for 2-heptanone was statistically significant different when compared with all the other ketones of the Table (P<0.05) | | | |

### Example 14

### The local anaesthetic effect of 3.4 mg/ml of 2-hexanone on the sciatic nerve

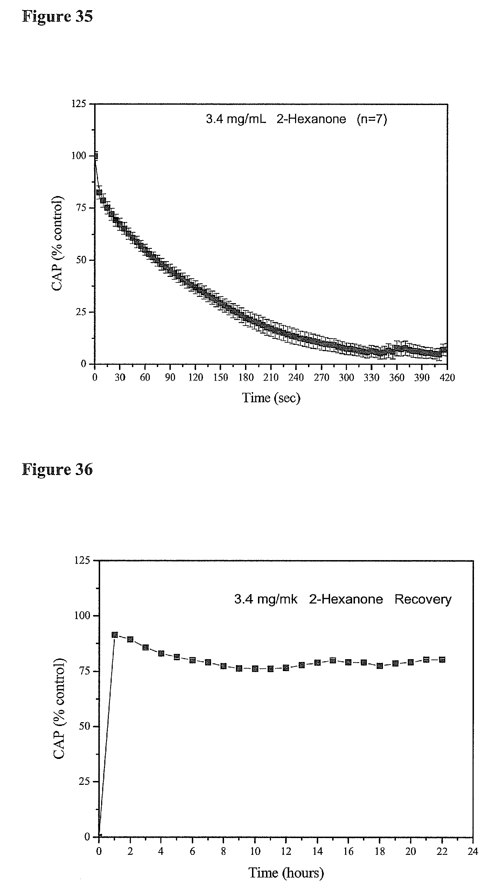

*Inhibition of the CAP:* The amplitude of the evoked nerve CAP decreased during incubation in 3.4 mg/mL 2-hexanone diluted in saline (see Figure 35). The CAP of 7 nerves was eliminated within 401.00±33.20 seconds (IT₀). The IT₅₀ was 74.42±0.79 seconds (n=7).
*Recovery of the CAP after wash:* The recovery was 83% in less than 1 h and remained at the level of 75% for 24h (Figure 36).

### Example 15

### The local anaesthetic effect of 3.4 mg/ml of 2-octanone on the sciatic nerve

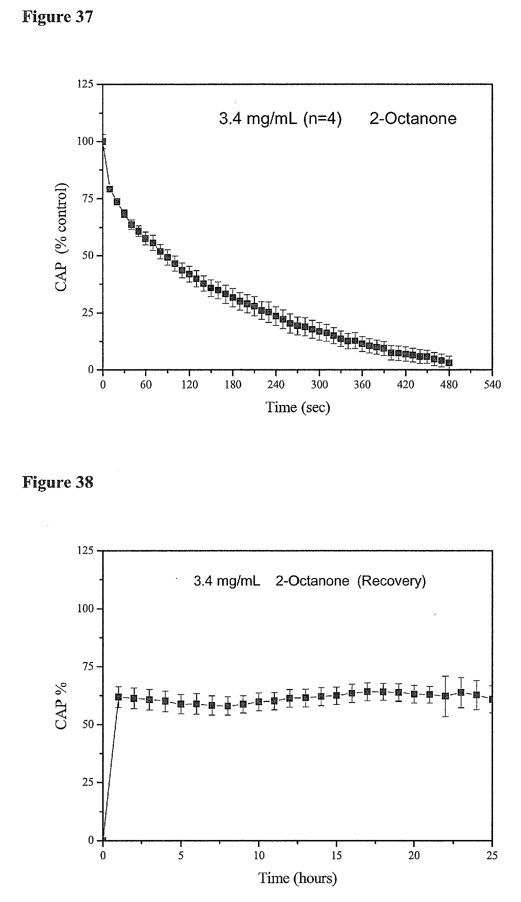

*Inhibition of the CAP:* The amplitude of the evoked CAP of the rat sciatic nerve decreased following incubation in 3.4 mg/ml 2-octanone in saline (see Figure 37). The nerve CAP of 4 nerves was eliminated within 555.00±75.00 seconds (IT₀). The IT₅₀ was 81.45±1.58 seconds (n=4).
*Recovery of the CAP after wash:* The recovery was 62 % at less than 1 h and remained at this level for 24 hours (Fig. 38).

### Example 16

### The local anaesthetic effect of 3.4 mg/ml of 3-heptanone on the sciatic nerve

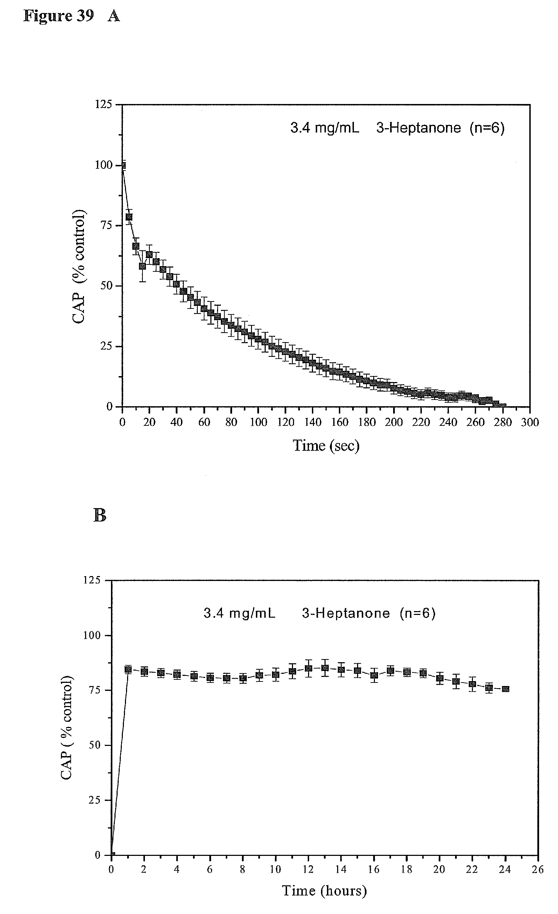

*Inhibition of the CAP:* The amplitude of the evoked nerve CAP decreased during incubation in 3.4 mg/ml 3-heptanone in saline (see Figure 39 A). For the 6 nerves examined the CAP was eliminated within 258.00±11.47 seconds (IT₀). The IT₅₀ was 40.82±1.67 seconds (n=6) (see Table 1).
*Recovery of the CAP after wash:* The recovery of the nerve CAP was 85 % of the control in less than 1 h and remained at this level for 24 hours (Figure 39 B).

### Example 17

### The local anaesthetic effect of 3.4 mg/ml of 2-pentanone and 3-pentanone on the sciatic nerve of the rat

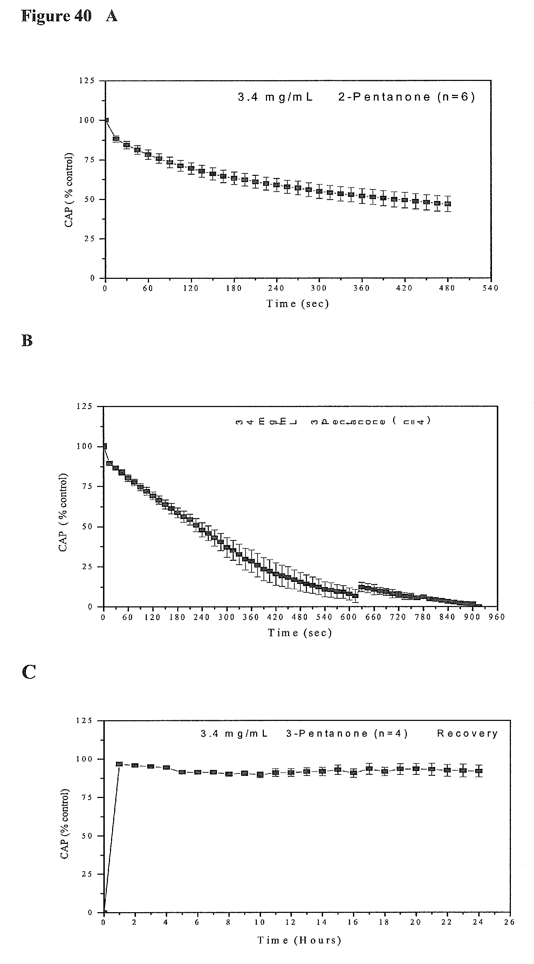

*2-pentanone-Inhibition of the CAP:* The amplitude of the evoked CAP of the rat sciatic nerve decreased following incubation in 3.4 mg/ml 2-pentanone in saline (see Figure 40A). The CAP was decreased to a level of 40-45% of the control within 400 seconds, but was never eliminated, thus it was not possible to estimate the IT₀. The IT₅₀ was 390.60 ± 0.10 seconds (n=6) (see Table 8).
*3-pentanone-Inhibition of the CAP:* The amplitude of the evoked CAP of the rat sciatic nerve decreased during incubation in 3.4 mg/ml 3-pentanone diluted in saline (see Figure 40 B). In four nerve preparations examined the CAP was eliminated within 765.00±86.60 seconds (IT₀). The IT₅₀ was 230.30±1.29 seconds (n=4) (see Table 8).
*Recovery of the CAP after wash:* The recovery of the CAP was 95 % of the control in less than 1 h after wash and remained at this level for 24 hours (Figure 40 C).

### Example 18

### The effects of 2-heptanone and lidocaine on ion channels expressed in mammalian cells

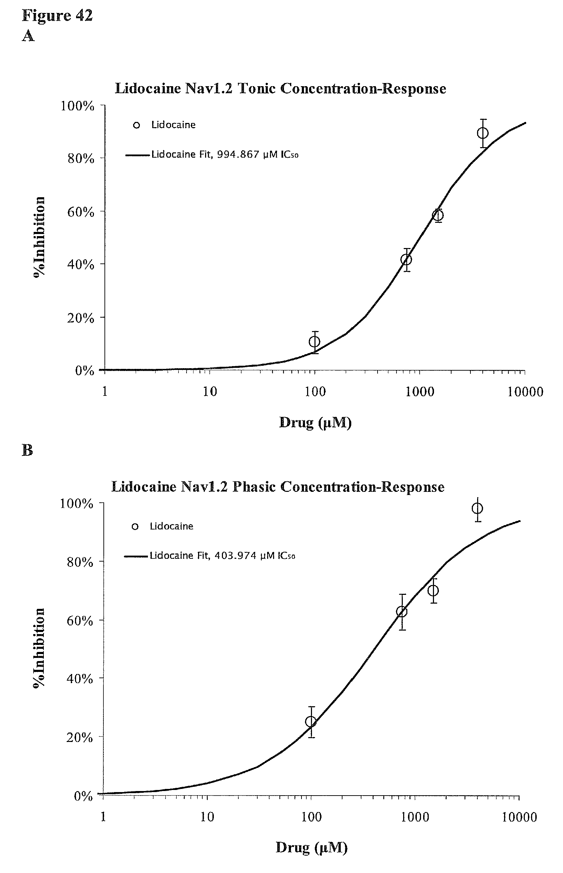

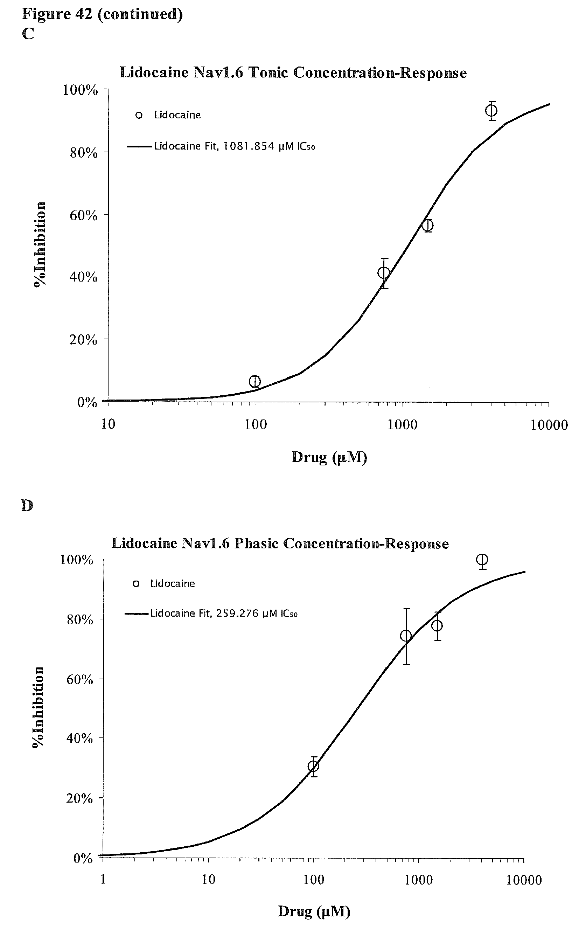

These data show that lidocaine and 2-heptanone share a similar pattern of action, inhibition, and recovery, suggesting that 2-heptanone also acts as an LA and may share a common target with lidocaine. Lidocaine blocks the voltage-gated sodium channels (VGNaCs) of mammals (Sheets, L.P et al 2011 Lidocaine reduces the transition to slow inactivation in Nav1.7 voltage-gated sodium channels. Br. J. Pharmacol. 164, 719-730). To test whether 2-heptanone can also act by blocking VGNaCs, we used the PatchXpress 7000A to expose mammalian CHO (Chinese hamster ovary) hNav1.2 and hNav1.6 channels to 2-heptanone and lidocaine under both tonic and phasic conditions (see Table 9 and Figures 41 and 42).

**Table 9: IC₅₀ (concentration required to inhibit function to 50% of normal) of 2-heptanone and lidocaine applied to hNav1.2 and hNav1.6 ion channels. Values are the mean IC₅₀ ± SEM. n = 6 in all cases.**

| | **hNav1.2 (IC₅₀)** | | **hNav1.6 (IC₅₀)** | |
|---|---|---|---|---|
| **Experiment** | **2-Heptanone** | **lidocaine** | **2-heptanone** | **lidocaine** |
| **Tonic** | 1.89 ± 0.01 mg/mL | 0.27 ± 0.01 mg/mL | 1.34 ± 0.01 mg/mL | 0.29 ± 0.01 mg/mL |
| **Phasic** | 1.28 ± 0.09 mg/mL | 0.10 ± 0.01 mg/mL | 0.82 ± 0.03 mg/mL | 0.07 ± 0.01 mg/mL |

### Conclusions

### 1) The local anesthetic action of 2-heptanone

The results shown above demonstrate that 2-heptanone has a local anaesthetic action, causing an inhibition of the nerve CAP of the isolated nerve and its fast recovery after washout. This effect is dose-dependent and occurs at concentrations such as 1.41 mg/mL (12.5 mM), 1.7 mg/mL (or 15.0 mM), 2.2 mg/mL (20.0 m), 3.4 mg/mL (30 mM) and even at 10 mg/mL (88.2 mM). The main parameters IT₅₀, IT₀ are summarised in Table 7 (Examples 1.2.3A,B, 4 A B, 5 A B, .8,9,10).

### 2) 2-heptanone vs lidocaine as local anesthetic on the isolated sciatic nerve ex-vivo

**a)** 2-heptanone can be considered as a good local anaesthetic for the peripheral nerves, since has almost identical action to lidocaine; a standard local anaesthetic with extensive use in clinical practice. Both compounds eliminate the nerve CAP within seconds, while a fast recovery, less than 1 h, occurred as soon as the compounds washout from the nerve-preparation. The comparison of the action, IT₅₀ and IT₀ of the two compounds, are shown in Table 7,
**b)** In more detail, at a concentration of 3.4 mg/mL 2-heptanone eliminated the nerve CAP with an IT₀ of 221.25 s while the IT₀ for lidocaine is 147.50 ± 9.96 s (Examples 8, 10 B and Example 11 A). The only difference between their action is that 2-heptanone eliminated the nerve CAP 70-75 seconds slower than lidocaine (Example 11A, Table 7). This is a minor time difference if someone consider that 2-heptanone and lidocaine were found to maintain local anaesthesia during intramuscular injection for over 2 h as the *in vivo* experiments have shown (Example 12).
c) As soon as the compounds, 2-heptanone and lidocaine, were washed out and replaced with normal saline there was a recovery of the nerve CAP to levels 87.5% of the control for 2-heptanone and 95.5% for lidocaine (no significant different P>0.05). The nerve CAP remained at this level for 24 h, indicating that both compounds have the same effect on the vitality of the nerve fibres (Example 8, 11A). This is also evidence that both compounds have no neurotoxic effect within the limits of 24 h.

### 3) 2-heptanone vs other local anesthetics using the isolated nerve preparation

2-heptanone can be compared to other standard local anaesthetics apart from lidocaine, like mevipacaine, aticaine and prilocaine, using the sciatic nerve preparation *(ex-vivo).* Within the time limit of 70 seconds of treatment, and with minimum and maximum effects fixed at 0 and 100% of control, 10.0 mg/ml of the other 3 local anesthetics decreased the nerve CAP to 5-17 % of the control, while 2-heptanone to 24% (Example 11 B curve e). 2-heptanone has an action about 5 to 7% higher than prilocaine, which is also extensively used in medical practice as a local anesthetic.

### 4) 2-heptanone vs lidocaine during intramuscular injection. In vivo study

***a)** In vivo,* intramuscular injection of 2-heptanone on the gastrocnemius muscle was found to have a similar effect as lidocaine (Example 12). The IT₅₀ values of 10.8 mg/kg of lidocaine and 7.2 mg/kg 2-heptanone were similar (*P* > 0.05), an indication that 2-heptanone was 1.5 times more effective than lidocaine when tested intramuscular on the gastrocnemius muscle.

The IT₅₀ value for 7.2 mg/kg of 2-heptanone was also measured for biceps and was estimated to be 9.13 seconds. There is a variation in the experiments since in the gastrocnemius muscle the IT₅₀ for the same amount of 2-heptanone was found to be longer, 25.78 seconds. This variation indicates that in this kind of experiment a significant role is played by the size of the muscle, the anaesthesia of the animal, the treatment with the Tween 80, the site of the injection of the compound and the even the region of the recording.

### b) Metabolism of lidocaine vs 2-heptanone

In the in vivo experiments mentioned above 2-3 h after the injection of 10.8 mg/kg lidocaine in the gastrocnemius muscle there was a gradual recovery of the mCAP to 70.3% (lidocaine) of the control. The elimination half-life of lidocaine following an intravenous bolus injection is typically 1.5 to 2 hours due to metabolism of the compound (S. IMAOKA et al. 1990, Lidocaine Metabolism by Human Cytochrome P-450s J.Pharm. Exper.Therap. Vol. 255, No. 3). Under identical conditions after the injection of 7.2 mg/kg 2-heptanone there was a 61% recovery of the mCAP, an indication that 2-heptanone is also metabolised in mammalians (Example 12).

### 5) 2-heptanone vs other ketones

Using the above methodology, we also compared the action of 2-heptanone with its other isomers, 3-heptanone, 4-heptanone and other alkyl-ketones (Examples 13-17). Heptanones had the faster inhibitory effect than other ketones. Amongst heptanones 2-heptanone had the fastest action (*P* < 0.05). The IT₀ for 2-heptanone was 221.88 s *(n* = 12, SEM ± 15.20), for 3-heptanone 258.00 (*n* = 6, SEM ± ±11.47), and for 4-heptanone 283.57 *(n* = 8, SEM ± 16.89s). In contrast, IT₀ was 401.03 s for 2-hexanone *(n* = 7, SEM ± 33.20), 555.00 s for 2-octanone (*n* = 4, SEM ± 75.00), 722.50 s for 3-octanone *(n* = 6, SEM ± 95.66), and greater than 20 h for 2-pentanone (*n* = 6). The recovery pattern monitored for 24 h was almost identical for all compounds (Examples 13-17).

The fastest action of 2-heptanone could be an answer to the following question: why do honeybees use 2-heptanone for paralysing small parasites and not another ketone? We assume that natural selection favoured 2-heptanone over similar compounds as it has the fastest inhibitory effect on the nervous system, as our results have shown. In the case of intruders, fast anesthesia provides an important defensive advantage.

### 6) 2-heptanone vs lidocaine on the Nav1.2 and Nav1.6 ion channels

2-heptanone and lidocaine both blocked the two channels that we tested. In the tonic experiments, 2-heptanone was 7 (for hNav1.2) and 4.6 (for hNav1.6) times less active than lidocaine. In the phasic experiments, 2-heptanone was 12.8 (for hNav1.2) and 11.7 (for hNav1.6) times less active than lidocaine. The hNav1.6 channel is located in the nodal area (Leterrier, C. et al. 2010, Voltage-gated sodium channel organization in neurons: Protein interactions and trafficking pathways. Neurosci. Lett. 486, 92-100) and is obviously involved in the inhibition of the action potential caused by LAs (Cummins, T.R. 2007 Setting up for the block: the mechanism underlying lidocaine's use-dependent inhibition of sodium channels. J. Phys. 582, 11).

However, there is a large discrepancy between the effects of 2-heptanone and lidocaine assessed with the patch-clamp of single ion channel and the whole nerve recordings from the sciatic nerve. The values of patch-clamp are indicative since at present, the situation appears to be more complex, since Nav1.6 is expressed alone or together with Nav1.1 in CNS and PNS nodes of Ranvier while Nav1.8 and Nav1.7 are expressed in the sensory neurons of the sciatic nerve. It is difficult to simultaneously monitor the interactive response of all individual Nav1 channels involved in generating action potentials; thus, we used the common *ex-vivo* method to investigate their overall response to the LA action of 2-heptanone and lidocaine, the sciatic nerve fibers of the rat.

The values of patch-clamp experiments are referred to a single ion channels, while the experiments on the whole nerve are concerned with the whole numbers of ion channels involved in the generation of the nerve action potential and we believe that are more reliable for the particular evaluation.

In terms of hNav1.2 and Nav1.6 ion channels, 2-heptanone appears to be less sensitive than lidocaine (4.6 to 12.8 fold). However, when 2-heptanone and lidocaine, were tested on whole nerve preparation, where real nerve fibres and ion channels are involved, the two compounds have the same pattern of action with 2-heptanone acting with 70-75 seconds or 1.54 fold slower.

### 7) The neurotoxicity of 2-heptanone vs the neurotoxicity of lidocaine

Thus the local anaesthetic activities of lidocaine and 2-heptanone are comparable. Within the time limit of the *ex-vivo* preparation used in this study, it was not possible to demonstrate any neurotoxic effects of either compounds. Moreover, in terms of neurotoxicity, 2-heptanone was found to have no neurotoxic effects on the peripheral nervous system of the rat using *in-vivo* experiments (Misumi, J. & Nagano, M., 1984, Neurophysiological studies on the relation between the structural properties and neurotoxicity of aliphatic hydrocarbon compounds in rats. Br. J. Ind. Med. 41, 526-532). In terms of toxicity 2-heptanone has an acute LD50 is 1760 mg/ kg, while for lidocaine the acute LD50 is 313 mg/kg (2-heptanone is 5.6 times less toxic than lidocaine). 2-heptanone can also be used without any risk of toxic side effects, since it is listed by the FDA as a "food additive" permitted for direct addition to food for human consumption. This is an indication that 2-heptanone appears to be less neurotoxic compared to the classical local anaesthetic lidocaine and can be used without any hesitation as a drug.

Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology or related fields are intended to be within the scope of the following claims.

## Claims

1. Heptanone or a salt thereof for use as a local anaesthetic.

2. A pharmaceutical composition comprising heptanone or a salt thereof and one or more pharmaceutically acceptable carriers, excipients or diluents, for use as a local anaesthetic.

3. A pharmaceutical composition for use according to claim 2, for administration by injection.

4. A pharmaceutical composition for use according to claim 3, for intramuscular or subcutaneous injection.

5. A pharmaceutical composition for use according to claim 2, for topical application.

6. A pharmaceutical composition for use according to any of claims 2 to 5, wherein the composition is in the form of a liquid.

7. A pharmaceutical composition for use according to claim 6, wherein the composition is in the form of an aqueous solution.

8. A pharmaceutical composition for use according to claim 7, wherein the composition comprises a saline solution.

9. A pharmaceutical composition for use according to claim 7 or claim 8, wherein the composition further comprises a buffer.

10. A pharmaceutical composition for use according to any of claims 7 to 9, wherein the solution is sterile.

11. A pharmaceutical composition for use according to any of claims 6 to 10, wherein the concentration of heptanone is from 10 to 30 mM.

12. Use of heptanone or a salt thereof in the preparation of a medicament for use as a local anaesthetic.

13. A pharmaceutical composition or use according to any preceding claim, wherein heptanone comprises 2-heptanone, 3-heptanone or 4-heptanone.

## Patentansprüche

1. Heptanon oder ein Salz davon für die Verwendung als Lokalanästhetikum.

2. Pharmazeutische Zusammensetzung, umfassend Heptanon oder ein Salz davon und einen oder mehrere pharmazeutisch verträgliche Träger, Hilfsstoffe oder Verdünnungsmittel, für die Verwendung als Lokalanästhetikum.

3. Pharmazeutische Zusammensetzung für die Verwendung gemäß Anspruch 2 für die Verabreichung durch Injektion.

4. Pharmazeutische Zusammensetzung für die Verwendung gemäß Anspruch 3 für die intramuskuläre oder subkutane Injektion.

5. Pharmazeutische Zusammensetzung für die Verwendung gemäß Anspruch 2 für die topische Anwendung.

6. Pharmazeutische Zusammensetzung für die Verwendung gemäß einem der Ansprüche 2 bis 5, wobei die Zusammensetzung in der Form einer Flüssigkeit vorliegt.

7. Pharmazeutische Zusammensetzung für die Verwendung gemäß Anspruch 6, wobei die Zusammensetzung in der Form einer wässrigen Lösung vorliegt.

8. Pharmazeutische Zusammensetzung für die Verwendung gemäß Anspruch 7, wobei die Zusammensetzung eine Kochsalzlösung umfasst.

9. Pharmazeutische Zusammensetzung für die Verwendung gemäß Anspruch 7 oder Anspruch 8, wobei die Zusammensetzung ferner einen Puffer umfasst.

10. Pharmazeutische Zusammensetzung für die Verwendung gemäß einen der Ansprüche 7 bis 9, wobei die Lösung steril ist.

11. Pharmazeutische Zusammensetzung für die Verwendung gemäß einen der Ansprüche 6 bis 10, wobei die Konzentration von Heptanon von 10 bis 30 mM beträgt.

12. Verwendung von Heptanon oder einem Salz davon bei der Herstellung eines Medikaments für die Verwendung als Lokalanästhetikum.

13. Pharmazeutische Zusammensetzung oder Verwendung gemäß einem derb vorherstehenden Ansprüche, wobei Heptanon 2-Heptanon, 3-Heptanon und 4-Heptanon umfasst.

## Revendications

1. Heptanone ou sel de celle-ci pour utilisation en tant qu'anesthésique local.

2. Composition pharmaceutique comprenant de l'heptanone ou un sel de celle-ci et un ou plusieurs véhicules, excipients ou diluants pharmaceutiquement acceptables, pour utilisation en tant qu'anesthésique local.

3. Composition pharmaceutique pour utilisation selon la revendication 2, pour administration par injection.

4. Composition pharmaceutique pour utilisation selon la revendication 3, pour injection intramusculaire ou sous-cutanée.

5. Composition pharmaceutique pour utilisation selon la revendication 2, pour application topique.

6. Composition pharmaceutique pour utilisation selon l'une quelconque des revendications 2 à 5, la composition étant sous la forme d'un liquide.

7. Composition pharmaceutique pour utilisation selon la revendication 6, la composition étant sous la forme d'une solution aqueuse.

8. Composition pharmaceutique pour utilisation selon la revendication 7, la composition comprenant une solution saline.

9. Composition pharmaceutique pour utilisation selon la revendication 7 ou la revendication 8, la composition comprenant en outre un tampon.

10. Composition pharmaceutique pour utilisation selon l'une quelconque des revendications 7 à 9, la solution étant stérile.

11. Composition pharmaceutique pour utilisation selon l'une quelconque des revendications 6 à 10, la concentration d'heptanone étant de 10 à 30 mM.

12. Utilisation d'heptanone ou un sel de celle-ci dans la préparation d'un médicament pour utilisation en tant qu'anesthésique local.

13. Composition pharmaceutique ou utilisation selon l'une quelconque des revendications précédentes, l'heptanone comprenant la 2-heptanone, la 3-heptanone ou la 4-heptanone.
